(19) **Europäisches Patentamt**
**European Patent Office**
**Office européen des brevets**

(11) **EP 4 062 824 A1**

(12) **EUROPEAN PATENT APPLICATION**
published in accordance with Art. 153(4) EPC

(43) Date of publication:
**28.09.2022 Bulletin 2022/39**

(21) Application number: **20903369.5**

(22) Date of filing: **15.12.2020**

(51) International Patent Classification (IPC):
**A61B 5/0205** (2006.01)

(86) International application number:
**PCT/CN2020/136538**

(87) International publication number:
**WO 2021/121226 (24.06.2021 Gazette 2021/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2019 CN 201911307688**

(71) Applicant: **Huawei Technologies Co., Ltd.**
**Shenzhen, Guangdong 518129 (CN)**

(72) Inventors:
• **LI, Luping**
**Shenzhen, Guangdong 518129 (CN)**

• **CHEN, Maolin**
**Shenzhen, Guangdong 518129 (CN)**
• **HAN, Yujia**
**Shenzhen, Guangdong 518129 (CN)**
• **JIA, Miao**
**Shenzhen, Guangdong 518129 (CN)**
• **GUO, Guangming**
**Shenzhen, Guangdong 518129 (CN)**

(74) Representative: **Pfenning, Meinig & Partner mbB**
**Patent- und Rechtsanwälte**
**Theresienhöhe 11a**
**80339 München (DE)**

(54) **ELECTROCARDIOSIGNAL PREDICTION METHOD AND DEVICE, TERMINALS, AND STORAGE MEDIUM**

(57) An electrocardiosignal prediction method and apparatus that are based on artificial intelligence (Artificial Intelligence, AI), a terminal (210, 320, 20), and a storage medium are provided, and are applicable to the field of vital sign recognition technologies. The method includes: obtaining an electrocardiosignal of a target user (S401); importing the electrocardiosignal into a preset atrial fibrillation signal classification model, to obtain a signal type, of the electrocardiosignal, output by the atrial fibrillation signal classification model (S402), where the atrial fibrillation signal classification model is obtained through training with an atrial fibrillation patient being a model training sample; and calculating, based on the signal type of the electrocardiosignal, a risk level of an atrial fibrillation occurrence, to predict whether the target user is to have an atrial fibrillation attack (S403). In this way, an atrial fibrillation event can be predicted. It is convenient for the user to determine a physical condition of the user, and a detection effect and user experience are improved.

FIG. 4

**Description**

This application claims priority to Chinese Patent Application No. 201911307688.7, filed with the China National Intellectual Property Administration on December 18, 2019 and entitled "ELECTROCARDIOSIGNAL PREDICTION METHOD AND APPARATUS, TERMINAL, AND STORAGE MEDIUM", which is incorporated herein by reference in its entirety.

**TECHNICAL FIELD**

This application pertains to the field of vital sign recognition technologies, and in particular, relates to an electrocardiosignal prediction method and apparatus, a terminal, and a storage medium.

**BACKGROUND**

Atrial fibrillation, abbreviated as A-fib, is the most common persistent chronic arrhythmia, which is usually caused by disorder atrial activities and irregular atrial compressions. As people pay more attention to health and an incidence of atrial fibrillation gradually increases, how to identify whether a user has atrial fibrillation becomes especially important. According to the existing atrial fibrillation detection technology, a user can be determined as an atrial fibrillation patient only when the user encounters an atrial fibrillation event. However, when the atrial fibrillation event occurs, life safety of the user may be endangered, and health of the user is affected. It can be learned that in the existing atrial fibrillation detection technology, only whether the user encounters an atrial fibrillation event can be announced, but the atrial fibrillation event of the user cannot be predicted. Therefore, a detection effect is poor.

**SUMMARY**

Embodiments of this application provide an electrocardiosignal prediction method and apparatus, a terminal, and a storage medium, to resolve a problem that an atrial fibrillation event of a user cannot be predicted and a detection effect is poor in the existing atrial fibrillation detection technology.

According to a first aspect, an embodiment of this application provides an electrocardiosignal prediction method, including:

  obtaining an electrocardiosignal of a target user;
  importing the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample; and
  calculating, based on the signal type of the electrocardiosignal, a risk level of an atrial fibrillation occurrence, to predict whether the target user is to have an atrial fibrillation attack.

In a possible implementation of the first aspect, before the importing the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample, the method further includes:

  obtaining a training signal set including a plurality of successively collected historical signals, where the training signal set includes at least one atrial fibrillation signal;
  extracting a risk signal other than the atrial fibrillation signal from the training signal set; and
  performing training on a preset original classification model by using the risk signal in the training signal set, to obtain the atrial fibrillation signal classification model.

In a possible implementation of the first aspect, the performing training on a preset original classification model by using the risk signal in the training signal set, to obtain the atrial fibrillation signal classification model includes:

  determining, based on a time difference between a collect time of the risk signal and a trigger time of an associated atrial fibrillation signal, a signal type corresponding to each risk signal;
  calculating a feature value of the risk signal in each preset signal feature dimension, to obtain a signal feature parameter of the risk signal; and
  performing training based on the signal feature parameter and the signal type, to obtain the atrial fibrillation signal classification model.

In a possible implementation of the first aspect, the obtaining a training signal set including a plurality of successively collected electrocardiosignals includes:

obtaining a motion parameter that is of the training user and that is obtained during collection of historical signals;
determining jitter duration of the historical signal;
determining, based on the jitter duration and the motion parameter, whether the historical signal is a valid signal; and
encapsulating all valid information based on an order of collect times of all valid signals, to obtain the training signal set.

In a possible implementation of the first aspect, the importing the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample includes:

determining a vital sign parameter of the target user based on the electrocardiosignal;
adjusting a classification threshold of the atrial fibrillation signal classification model based on the vital sign parameter; and
identifying the signal type of the electrocardiosignal by using an adjusted atrial fibrillation signal classification model.

In a possible implementation of the first aspect, the calculating, based on the signal type of the electrocardiosignal, a risk level of an atrial fibrillation occurrence, to predict whether the target user is to have an atrial fibrillation attack includes:

counting a quantity of electrocardiosignals that are of the target user and whose signal types are a risk type within a preset time period; and
calculating an atrial fibrillation occurrence probability based on the signal quantity.

In a possible implementation of the first aspect, if the atrial fibrillation occurrence probability is greater than a preset probability threshold, after the calculating, based on the signal type of the electrocardiosignal, a risk level of atrial fibrillation occurrence, to predict whether the target user is to have an atrial fibrillation attack, the method further includes:

determining an associated user preset by the target user, and sending warning information to a terminal of the associated user; and/or
obtaining, based on current location information of the target user, an address of a hospital closest to a location in the location information; and
generating a path for treatment based on the location information and the hospital address.

In a possible implementation of the first aspect, after the calculating, based on the signal type of the electrocardiosignal, a risk level of an atrial fibrillation occurrence, to predict whether the target user is to have an atrial fibrillation attack, the method further includes: if a new electrocardiosignal is received, identifying a new signal type of the new electrocardiosignal by using the atrial fibrillation signal classification model;

recalculating an atrial fibrillation occurrence probability of the target user based on the new signal type; and

generating an atrial fibrillation probability curve based on all identified atrial fibrillation occurrence probabilities.

According to a second aspect, an embodiment of this application provides an electrocardiosignal prediction apparatus, including:

an electrocardiosignal obtaining unit, configured to obtain an electrocardiosignal of a target user;
a signal type identification unit, configured to import the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample; and
an atrial fibrillation occurrence probability calculation unit, configured to calculate, based on the signal type of the electrocardiosignal, a risk level of an atrial fibrillation occurrence, to predict whether the target user is to have an atrial fibrillation attack.

According to a third aspect, an embodiment of this application provides a terminal device, including a memory, a processor, and a computer program that is stored in the memory and that is run on the processor. When executing the computer program, the processor implements the electrocardiosignal prediction method according to any one of the implementations of the first aspect.

According to a fourth aspect, an embodiment of this application provides a computer-readable storage medium. The computer-readable storage medium stores a computer program, and when the computer program is executed by a processor, the electrocardiosignal prediction method according to any one of the implementations of the first aspect is implemented.

According to a fifth aspect, an embodiment of this application provides a computer program product. When the computer program product is run on a terminal device, the terminal device is enabled to perform the electrocardiosignal prediction method according to any one of the implementations of the first aspect.

It may be understood that, for beneficial effects of the second aspect to the fifth aspect, refer to the related descriptions in the first aspect. Details are not described herein again.

Compared with the conventional technology, embodiments of this application have the following beneficial effects.

In embodiments of this application, a signal type of each electrocardiosignal is determined by importing a collected electrocardiosignal into the preset atrial fibrillation signal classification model, signal types of electrocardiosignals of the user in a plurality of collect periods are obtained, the atrial fibrillation occurrence probability of the target user is calculated based on the signal types of the plurality of electrocardiosignals, and an atrial fibrillation event is predicted based on a value of the atrial fibrillation occurrence probability. Therefore, it is convenient for the user to determine a physical condition of the user, and a detection effect and user experience are improved.

**BRIEF DESCRIPTION OF DRAWINGS**

FIG. 1 is a block diagram of a partial structure of a mobile phone according to an embodiment of this application;
FIG. 2 is a block diagram of a structure of an electrocardiosignal prediction system according to an embodiment of this application;
FIG. 3 is a block diagram of a structure of an electrocardiosignal prediction system according to another embodiment of this application;
FIG. 4 is an implementation flowchart of an electrocardiosignal prediction method according to a first embodiment of this application;
FIG. 5 is a schematic diagram of outputting an atrial fibrillation occurrence probability according to an embodiment of this application;
FIG. 6 is a schematic diagram of comparison between related markers of an atrial fibrillation patient and a common patient according to an embodiment of this application;
FIG. 7 is a specific implementation flowchart of an electrocardiosignal prediction method according to a second embodiment of this application;
FIG. 8 is a schematic diagram of a training signal set according to an embodiment of this application;
FIG. 9 is a specific implementation flowchart of S703 in an electrocardiosignal prediction method according to a third embodiment of this application;
FIG. 10 is a schematic diagram of a marker of a signal type according to an embodiment of this application;
FIG. 11 is a specific implementation flowchart of S4022 in an electrocardiosignal prediction method according to a fourth embodiment of this application;
FIG. 12 is a specific implementation flowchart of S402 in an electrocardiosignal prediction method according to a fifth embodiment of this application;
FIG. 13 is a specific implementation flowchart of S403 in an electrocardiosignal prediction method according to a sixth embodiment of this application;
FIG. 14 is a specific implementation flowchart of an electrocardiosignal prediction method according to a seventh embodiment of this application;
FIG. 15 is a schematic diagram of outputting warning information according to an embodiment of this application;
FIG. 16 is a schematic diagram of outputting a path for treatment according to an embodiment of this application;
FIG. 17 is a specific implementation flowchart of an electrocardiosignal prediction method according to an eighth embodiment of this application;
FIG. 18 is a schematic diagram of outputting an atrial fibrillation probability curve according to an embodiment of this application;
FIG. 19 is a block diagram of a structure of an electrocardiosignal prediction device according to an embodiment of this application; and

FIG. 20 is a schematic diagram of a terminal device according to another embodiment of this application.

**DESCRIPTION OF EMBODIMENTS**

In the following descriptions, to illustrate rather than limit, specific details such as a particular system structure and a technology are provided to make a thorough understanding of embodiments of this application. However, a person skilled in the art should know that this application may also be implemented in other embodiments without these specific details. In other cases, detailed descriptions of well-known systems, apparatuses, circuits, and methods are omitted, so that this application is described without being obscured by unnecessary details.

It should be understood that, when used in the specification and claims of this application, the term "include" indicates presence of described features, entireties, steps, operations, elements, and/or components, but does not exclude presence or addition of one or more other features, entireties, steps, operations, elements, components, and/or collections thereof.

It should be further understood that the term "and/or" used in the specification and the claims of this application indicates any combination and all possible combinations of one or more items listed in association, and includes the combinations.

As used in the specification and the claims of this application, the term "if' may be interpreted as "when", "once", "in response to determining", or "in response to detecting" depending on the context. Similarly, the phrase "if it is determined" or "if [the described condition or event] is detected" may be interpreted as "once determined", "in response to determining", "once [the described condition or event] is detected", or "in response to detecting [the described condition or event]" depending on the context.

In addition, in the descriptions of the specification and the claims of this application, the terms "first", "second", "third", and the like are merely used for differentiation and description, but shall not be understood as an indication or an implication of relative importance.

Referring to "an embodiment" or "some embodiments" or the like in the specification of this application means that one or more embodiments of this application include a specific feature, structure, or characteristic described with reference to the embodiment. Therefore, statements such as "in an embodiment", "in some embodiments", "in some other embodiments", and "in other embodiments" that appear at different places in this specification do not necessarily mean referring to a same embodiment, instead, they mean "one or more but not all of the embodiments", unless otherwise specifically emphasized. The terms "include", "contain", "have", and their variants all mean "include but are not limited to", unless otherwise specifically emphasized.

An electrocardiosignal prediction method provided in embodiments of this application may be applied to a terminal device such as a mobile phone, a tablet computer, a wearable device, an in-vehicle device, an augmented reality (augmented reality, AR) device/a virtual reality (virtual reality, VR) device, a notebook computer, an ultra-mobile personal computer (ultra-mobile personal computer, UMPC), a netbook, or a personal digital assistant (personal digital assistant, PDA), and may be applied to a database, a server, and a service response system that is based on terminal artificial intelligence. A specific type of the terminal device is not limited in embodiments of this application.

For example, the terminal device may be a station (STAION, ST) in a WLAN, a cellular phone, a cordless phone, a session initiation protocol (Session Initiation Protocol, SIP) phone, a wireless local loop (Wireless Local Loop, WLL) station, a personal digital assistant (Personal Digital Assistant, PDA) device, a handheld device having a wireless communication function, a computing device or another processing device connected to a wireless modem, a computer, a laptop computer, a handheld communications device, a handheld computing device, and/or another device for communicating in a wireless system and a next-generation communications system, for example, a mobile terminal in a 5G network or a mobile terminal in a future evolved public land mobile network (Public Land Mobile Network, PLMN).

By way of example but not limitation, when the terminal device is a wearable device, the wearable device may alternatively be a generic term for wearable devices such as glasses, gloves, watches, clothes, and shoes that are developed based on intelligent design of daily wearing by using wearable technologies. The wearable device is a portable device that is directly worn on a body or integrated into a cloth or an accessory of a user, and is attached to the user to collect an atrial fibrillation signal of the user. The wearable device not only is a hardware device, but also implements a powerful function through software support, data exchange, and cloud interaction. In a broad sense, the wearable intelligent device includes a device that provides a complete function, has a large size, and can implement all or some functions without relying on a smartphone, for example, a smart watch or smart glasses; and includes a device that focuses only on a specific type of application and needs to be used in combination with another device such as a smartphone, for example, various smart bands and smart jewelry used for vital sign monitoring.

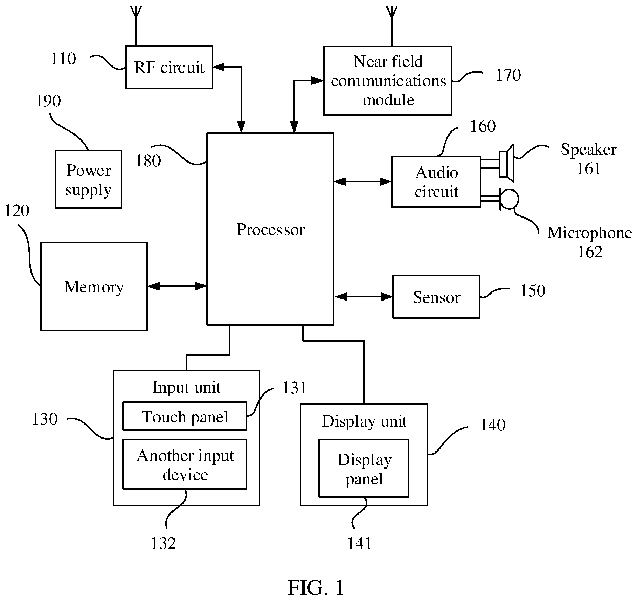

For example, the terminal device is a mobile phone. FIG. 1 is a block diagram of a partial structure of the mobile phone according to an embodiment of this application. Refer to FIG. 1. The mobile phone includes components such as a radio frequency (Radio Frequency, RF) circuit 110, a memory 120, an input unit 130, a display unit 140, a sensor 150, an audio circuit 160, a near field communications module 170, a processor 180, and a power supply 190. A person skilled in the art may understand that the structure of the mobile phone shown in FIG. 1 does not constitute a limitation on the mobile phone. The mobile phone may include more or fewer components than those shown in the figure, or may include a combination of some components, or may include different component arrangements.

The following describes each component of the mobile phone in detail with reference to FIG. 1.

The RF circuit 110 may be configured to receive and send a signal in an information receiving or sending process or during a call. Particularly, after receiving downlink information from a base station, the RF circuit 110 sends the downlink information to the processor 180 for processing, and in addition, sends related uplink data to the base station. Usually, an RF circuit includes but is not limited to including an antenna, at least one amplifier, a transceiver, a coupler, a low noise amplifier (Low Noise Amplifier, LNA), a duplexer, and the like. In addition, the RF circuit 110 may further communicate with a network and another device through wireless communication. Any communications standard or protocol may be used for wireless communication, including but not limited to a global system for mobile communication (Global System for Mobile Communication, GSM), a general packet radio service (General Packet Radio Service, GPRS), code division multiple access (Code Division Multiple Access, CDMA), wideband code division multiple access (Wideband Code Division Multiple Access, WCDMA), long term evolution (Long Term Evolution, LTE), an email, a short message service (Short Message Service, SMS), and the like. An atrial fibrillation signal of a user fed back by another terminal is received through the RF circuit 110.

The memory 120 may be configured to store a software program and a module. The processor 180 runs the software program and the module stored in the memory 120 to perform various functional applications of the mobile phone and data processing, for example, store a received atrial fibrillation signal in the memory 120. The memory 120 may mainly include a program storage area and a data storage area. The program storage area may store an operating system, an application program required by at least one function (for example, a sound playing function or an image playing function), and the like. The data storage area may store data (for example, audio data or an address book) and the like created based on use of the mobile phone. In addition, the memory 120 may include a high-speed random access memory, or may further include a nonvolatile memory, such as at least one magnetic disk storage device, a flash memory device, or another volatile solid-state storage device.

The input unit 130 may be configured to: receive entered digital or character information, and generate key signal input related to a user setting and function control of the mobile phone 100. Specifically, the input unit 130 may include a touch panel 131 and another input device 132. The touch panel 131, also referred to as a touchscreen, may collect a touch operation of a user on or near the touch panel 131 (such as an operation of the user on or near the touch panel 131 by using any suitable object or attachment, such as a finger or a touch pen), and drive a corresponding connection apparatus according to a preset program.

The display unit 140 may be configured to display information input by the user, information provided for the user, and various menus of the mobile phone, for example, output a received atrial fibrillation signal of the user, and output an identification result after a type of the atrial fibrillation signal is determined. The display unit 140 may include a display panel 141. Optionally, the display panel 141 may be configured in a form of a liquid crystal display (Liquid Crystal Display, LCD), an organic light-emitting diode (Organic Light-Emitting Diode, OLED), or the like. Further, the touch panel 131 may cover the display panel 141. When detecting a touch operation on or near the touch panel 131, the touch panel 131 transfers the touch operation to the processor 180 to determine a type of the touch event, and then the processor 180 provides corresponding visual output on the display panel 141 based on the type of the touch event. In FIG. 1, the touch panel 131 and the display panel 141 are used as two independent components to implement input and input functions of the mobile phone. However, in some embodiments, the touch panel 131 and the display panel 141 may be integrated to implement the input and output functions of the mobile phone.

The mobile phone 100 may further include at least one type of sensor 150, for example, a light sensor, a motion sensor, and another sensor. Specifically, the light sensor may include an ambient light sensor and a proximity sensor. The ambient light sensor may adjust luminance of the display panel 141 based on brightness of ambient light, and the proximity sensor may turn off the display panel 141 and/or backlight when the mobile phone approaches an ear of the user. As a type of motion sensor, an accelerometer sensor may detect a value of acceleration in each direction (usually on three axes), may detect a value and a direction of gravity when the accelerometer sensor is in a stationary state, and may be used in an application for identifying a mobile phone posture (such as screen switching between a landscape mode and a portrait mode, a related game, or magnetometer posture calibration), a function related to vibration identification (such as a pedometer or a knock), or the like. Other sensors such as a gyroscope, a barometer, a hygrometer, a thermometer, or an infrared sensor may be further configured in the mobile phone. Details are not described herein. Further, if the terminal device is configured to collect the atrial fibrillation signal of the user, an electrocardiosignal sensor may be further disposed on the terminal device. By using the electrocardiosignal sensor, an electrocardiosignal of the user is obtained.

The audio circuit 160, a speaker 161, and a microphone 162 may provide an audio interface between the user and the mobile phone. The audio circuit 160 may transmit, to the speaker 161, an electrical signal that is converted from received audio data, and the speaker 161 converts the electrical signal into a sound signal for output. In addition, the microphone 162 converts a collected sound signal into an electrical signal, and the audio circuit 160 receives the electrical signal, converts the electrical signal into audio data, and outputs the audio data to the processor 180 for processing. Processed audio data is sent to, for example, another mobile phone by using the RF circuit 110, or the audio data is output to the memory 120 for further processing. For example, the terminal device may play a prediction result of the electrocardiosignal by using the audio circuit 160, and notify the user by using a voice signal.

The terminal device may receive, by using the near field communications module 170, an atrial fibrillation signal sent by another device. For example, the near field communications module 170 is integrated with a Bluetooth communications module, establishes a communication connection to a wearable device by using the Bluetooth communications module, and receives an atrial fibrillation signal fed back by the wearable device. Although FIG. 1 shows the near field communications module 170, it may be understood that the near field communications module 170 is not a mandatory component of the mobile phone 100, and the near field communications module 170 may be omitted as required, provided that the scope of the essence of this application is not changed.

The processor 180 is a control center of the mobile phone, and uses various interfaces and lines to connect all parts of the entire mobile phone. By running or executing the software program and/or the module stored in the memory 120 and invoking data stored in the memory 120, the processor 180 performs various functions of the mobile phone and data processing, to perform overall monitoring on the mobile phone. Optionally, the processor 180 may include one or more processing units. Preferably, the processor 180 may integrate an application processor and a modem processor. The application processor mainly processes an operating system, a user interface, an application, and the like. The modem processor mainly processes wireless communication. It may be understood that the foregoing modem processor may be not integrated into the processor 180.

The mobile phone 100 further includes the power supply 190 (for example, a battery) supplying power to the components. Preferably, the power supply may be logically connected to the processor 180 by using a power management system, to implement functions such as charging and discharging management and power consumption management by using the power management system.

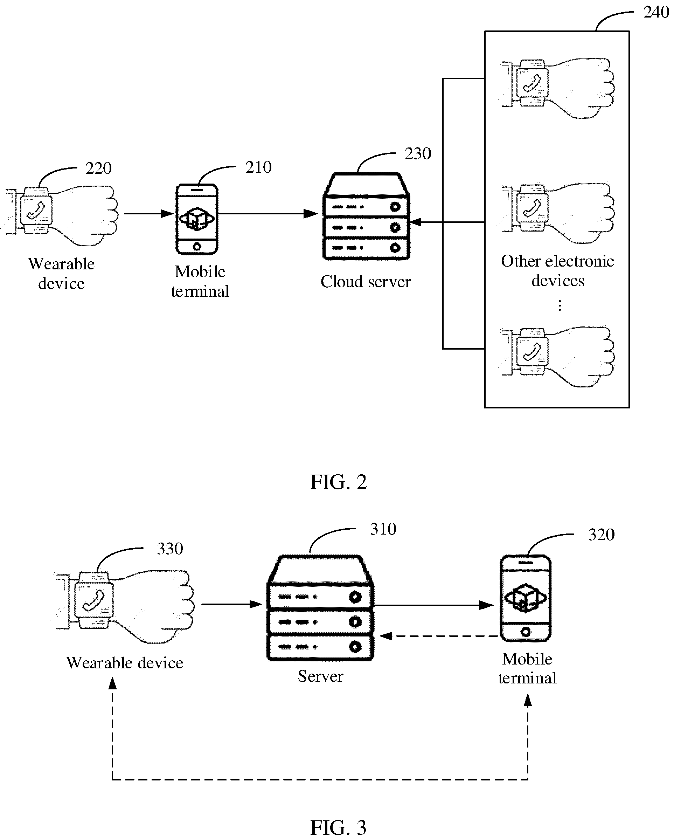

FIG. 2 is a block diagram of a structure of an electrocardiosignal prediction system according to an embodiment of this application. Refer to FIG. 2. The electrocardiosignal prediction system includes a mobile terminal 210 and a wearable device 220. The mobile terminal 210 may establish a communication connection to the wearable device 220 through near field communication.

An electrocardiosignal prediction apparatus provided in this application is specifically the mobile terminal 210 used by a user. The mobile terminal 210 may receive an electrocardiosignal sent by the wearable device 220. For example, the mobile terminal 210 establishes the communication connection to the wearable device 220 through near field communication, such as Bluetooth communication or Wi-Fi communication, and receives an electrocardiosignal sent by the wearable device 220. The mobile terminal 210 may further communicate with another remote communications terminal that stores an electrocardiosignal of a target user through wired communication or wireless communication, and receives an electrocardiosignal sent by the remote communications terminal. The mobile terminal 210 configures an atrial fibrillation signal classification model by receiving electrocardiosignals of a plurality of different users, and identifies, based on the atrial fibrillation signal classification model, signal types of electrocardiosignals fed back by the wearable device 220 in a plurality of collect periods. In addition, an atrial fibrillation occurrence probability of the target user is calculated based on the signal types of the plurality of electrocardiosignals, to predict an atrial fibrillation event of the user.

The wearable device 220 is specifically configured to collect a biometric feature signal of the user. The biometric feature signal may be an originally collected electrocardiosignal, or may be an atrial fibrillation signal generated through processing by using an atrial fibrillation identification algorithm. If the electrocardiosignal is collected, the original electrocardiosignal may be sent to the mobile terminal 210. The mobile terminal 210 performs atrial fibrillation identification on the electrocardiosignal, and converts the electrocardiosignal into an atrial fibrillation signal. Alternatively, a processing module disposed in the wearable device 220 may convert the electrocardiosignal into an atrial fibrillation signal, and then send the atrial fibrillation signal to the mobile terminal 210.

Preferably, the electrocardiosignal prediction system further includes a cloud server 230. The cloud server may receive an electrocardiosignal fed back by each of other electronic devices 240. Particularly, the electronic device 240 feeds back an electrocardiosignal of an atrial fibrillation patient. The cloud server constructs the foregoing atrial fibrillation signal classification model based on the electrocardiosignal of the atrial fibrillation patient, and releases the foregoing atrial fibrillation signal classification model to each terminal device 210. The terminal device 210 may download the classification model from the cloud server 230, and does not need to construct a model locally.

FIG. 3 is a block diagram of a structure of an electrocardiosignal prediction system according to another embodiment of this application. Refer to FIG. 3. The electrocardiosignal prediction system includes a server 310, a terminal device 320, and a wearable device 330. The server 310, the terminal device 320, and the wearable device 330 may communicate with each other through a wired network and/or a wireless network.

The server 310 may receive atrial fibrillation signals sent by a plurality of wearable devices 330, that is, the server is a cloud device. Optionally, if the wearable device 330 cannot access the Internet, in this case, the wearable device 330 may establish a communication connection to the terminal device 320 through near field communication. In this case, a client program associated with the server 310 may be installed in the terminal device 320. The wearable device 330 sends an electrocardiosignal to the terminal device 320 for storage, and when the terminal device 320 runs the client program, the terminal device 320 encapsulates the electrocardiosignal by using the client program, and sends an encapsulated data packet to the server 310. After receiving the electrocardiosignal sent by the wearable device, the server may identify a signal type of the electrocardiosignal by using a built-in atrial fibrillation signal classification model, and feed back the signal type to the terminal device 320 corresponding to a target user or send the signal type to the wearable device 330. Certainly, if the server 310 configures corresponding databases for different users, the server 310 may store, based on a user identifier of the electrocardiosignal, the electrocardiosignal into a database associated with the target user, calculate an atrial fibrillation occurrence probability of the target user based on signal types of all historical signals of the target user in the database, and return the atrial fibrillation occurrence probability to the wearable device 330 or the terminal device 320 for display and output.

After receiving a signal type of a currently collected electrocardiosignal of the target user, the terminal device 320 may determine an atrial fibrillation occurrence probability of the target user based on signal types of all collected electrocardiosignals, and output the atrial fibrillation occurrence probability by using a display interface. The terminal device 320 may be further provided with a communications module, for example, a Bluetooth communications module or a Wi-Fi communications module. The terminal device 320 receives, by using the communications module, the electrocardiosignal fed back by the wearable device 330 that is used by the user, and sends the atrial fibrillation signal to the server 310 by using the client program installed locally. Optionally, an atrial fibrillation occurrence probability conversion model is installed in the client program of the terminal device 320. The terminal device 320 generates a signal type sequence based on an order of collect times of the signal types of all the collected electrocardiosignals, imports the signal type sequence into the conversion model, and calculates the atrial fibrillation occurrence probability of the target user.

It should be noted that the server 310 may store a correspondence between the wearable device 330 and the terminal device 320. The wearable device 330 and the terminal device 320 that have a correspondence may belong to a same entity user. That is, the user may collect the electrocardiosignal by using the wearable device 330, and upload the electrocardiosignal to the server 310. After calculating the signal type corresponding to the electrocardiosignal or calculating the atrial fibrillation occurrence probability based on the signal types of the plurality of electrocardiosignals, the server 310 feeds back an output result to the terminal device 320 for display. Certainly, if the target user has an associated user, the server 310 may send the foregoing output result to the target user and a terminal device 320 of the associated user.

The wearable device 330 may be an intelligent wearable device, for example, a smart band or a smart watch, and a client program that matches the server 310 may be installed in the wearable device. After collecting the electrocardiosignal of the user, the wearable device sends the collected signal to the server 310 by using the client program. Certainly, the wearable device 330 may send the electrocardiosignal to the terminal device 320 that is in a same environment as the wearable device 330, to upload the electrocardiosignal to the server 310 by using the terminal device 320.

By way of example but not limitation, the target user is an elderly person, and the associated user of the target user is a child of the elderly person. The wearable device 330 is worn on a wrist of the elderly person. After collecting an electrocardiosignal of the elderly person, the wearable device 330 may directly send the electrocardiosignal to the server 310 by using a built-in communications module, or send the electrocardiosignal to the terminal device 320 of the elderly person, and the terminal device 320 forwards the electrocardiosignal to the server 310. After receiving the electrocardiosignal, the server 310 may identify a signal type of the electrocardiosignal by using the atrial fibrillation signal classification model, and may directly feed back the signal type to the terminal device 320 of the elderly person, or may send an atrial fibrillation occurrence probability to the terminal device 320 of the elderly person and the terminal device 320 of the child of the elderly person after calculating the atrial fibrillation occurrence probability of the elderly person. Preferably, when detecting that a value of the atrial fibrillation occurrence probability of the elderly person is greater than a preset probability threshold, the server 310 sends the atrial fibrillation occurrence probability of the elderly person to the terminal device 320 of the child of the elderly person. After receiving the atrial fibrillation occurrence probability, the terminal device 320 may perform output by using an interaction model, to notify the elderly person of a current physical condition.

In embodiments of this application, a process is performed by a device on which an electrocardiosignal prediction program is installed. By way of example but not limitation, the device on which the electrocardiosignal prediction program is installed may be specifically a terminal device. The terminal device may be a smartphone, a tablet computer, a notebook computer, or the like used by a user. The terminal device identifies a type of an obtained electrocardiosignal of a target user, and determines an atrial fibrillation occurrence probability of the target user. FIG. 1 is an implementation flowchart of an electrocardiosignal prediction method according to a first embodiment of this application.

S401. Obtain an electrocardiosignal of a target user.

In this embodiment, the electrocardiosignal may be an electrocardiosignal obtained by a wearable device or an electrocardiosignal collection device. An electrocardiosignal sensor, for example, an electrocardiogram (Electrocardiogram, ECG) sensor or a photoplethysmograph (Photoplethysmograph, PPG) sensor, may be disposed on the wearable device or the electrocardiosignal collection device, and may be configured to obtain an electrocardiosignal of a user wearing the device or a detected user. The wearable device may be a device that can be in contact with skin of the user, such as a wristband or a watch. The device may obtain a heart rate value of the user wearing the device by detecting a blood vessel expansion status in a contact area, and generate an electrocardiosignal of the user based on a heart rate value corresponding to each collecting moment. Certainly, if the terminal device is provided with an electrocardiosignal sensor, the electrocardiosignal of the user may be collected by the electrocardiosignal sensor. Optionally, after obtaining the electrocardiosignal, the terminal device may preprocess the electrocardiosignal by using a preset signal optimization algorithm, so that accuracy of subsequent type identification can be improved. An optimization manner includes but is not limited to one or a combination of the following: signal amplification, signal filtering, abnormality detection, signal recovery, and the like.

Abnormality detection is specifically extracting a plurality of waveform feature parameters such as maximum signal continuity duration, a quantity of waveform interruptions, collection interruption duration, and a waveform signal-to-noise ratio based on a collected signal waveform of an original electrocardiosignal, and calculating signal quality of the electrocardiosignal based on the collected waveform feature values. If it is detected that the signal quality is lower than a valid signal threshold, the electrocardiosignal is identified as an invalid signal, and a subsequent signal type identification operation is not performed on the invalid signal. On the contrary, if the signal quality is higher than the valid signal threshold, the electrocardiosignal is identified as a valid signal, and S402 and S403 are performed.

Signal recovery is specifically performing waveform fitting on an interruption region in an electrocardiosignal collection process by using a preset waveform fitting algorithm, to generate a continuous electrocardiosignal waveform. The waveform fitting algorithm may be a neural network, and a parameter in the waveform fitting algorithm is adjusted by collecting a historical signal of the target user, so that a fitted electrocardiosignal waveform trend matches a waveform trend of the target user. In this way, a waveform fitting effect is improved. Preferably, the signal recovery operation is performed after the abnormality detection operation because when a missing waveform of the electrocardiosignal is modified by using a signal, collection quality of the electrocardiosignal is improved, and consequently the abnormality detection operation is affected, and an abnormal signal whose collection quality is poor cannot be identified. Therefore, the terminal device may first determine, by using an abnormality detection algorithm, whether the electrocardiosignal is a valid signal. If the electrocardiosignal is the valid signal, signal recovery is performed on the electrocardiosignal by using a signal recovery algorithm. On the contrary, if the electrocardiosignal is an abnormal signal, signal recovery does not need to be performed. In this way, an unnecessary recovery operation is reduced.

In a possible implementation, a storage unit may be disposed in the wearable device or the electrocardiosignal collection device. The foregoing two devices each store the collected electrocardiosignal of the user in the storage unit. When it is detected that the electrocardiosignal stored in the storage unit meets a preset upload threshold, for example, when a data volume of the electrocardiosignal is greater than a preset data volume threshold, or when collect duration of the electrocardiosignal meets a corresponding upload threshold, the stored electrocardiosignal may be encapsulated and sent to the terminal device.

Optionally, in this embodiment, the terminal device may receive the electrocardiosignal of the target user from another device or obtain the electrocardiosignal of the target user by using a built-in sensor. If the electrocardiosignal of the target user is obtained by using another device, for example, a wearable device or an electrocardiosignal collection device, corresponding electrocardiosignal feedback periods may be configured for the different collection devices. The collection device may periodically send an atrial fibrillation signal of the user to the terminal device based on the feedback period. For example, if a length of the collect period is 48 seconds, the collection device may generate one signal segment based on the collected electrocardiosignal with the collect period being 48 seconds, and send one electrocardiosignal segment to the terminal device every 48 seconds. The terminal device may identify a signal type corresponding to each electrocardiosignal segment. Optionally, if a client program corresponding to the terminal device is configured for the collection device, when the user starts the client program, an electrocardiosignal collected between a moment at which a previous electrocardiosignal is fed back and a current startup moment of the client program may be encapsulated by using the client program, and the electrocardiosignal between the two moments is sent to the terminal device. The terminal device collectively performs type identification on all electrocardiosignals. The wearable device does not need to establish a persistent connection to the terminal device for a long time. A communication link between the wearable device and the terminal device is established only when the client program is started. Therefore, energy consumption of the electrocardiosignal collection apparatus and the terminal device is reduced, and a battery life of the device is improved.

S402. Import the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample.

In this embodiment, the atrial fibrillation signal classification model is preset in the terminal device, and the collected electrocardiosignal is imported into the atrial fibrillation signal classification model, so that the signal type corresponding to the electrocardiosignal can be determined. The signal type may be used to indicate an occurrence probability of an atrial fibrillation event. By way of example but not limitation, the terminal device may classify electrocardiosignals into two types: a first-type signal and a second-type signal. The first-type signal indicates that there is a high risk of an occurrence of an atrial fibrillation event, and the second-type signal indicates that there is a low probability of an occurrence of an atrial fibrillation event for the user. Certainly, different levels may be classified by the terminal device based on the probability of the occurrence of the atrial fibrillation event, and different levels correspond to one signal type. For example, atrial fibrillation occurrence probabilities are classified into N levels. A higher level indicates a lower probability of an occurrence of a corresponding atrial fibrillation event, and a lower level indicates a higher probability of an occurrence of a corresponding atrial fibrillation event. In this case, the $N^{th}$ level corresponds to the lowest atrial fibrillation occurrence probability, and an atrial fibrillation occurrence probability corresponding to the first level is the highest. The terminal device may identify, based on the foregoing atrial fibrillation classification model, a level to which the electrocardiosignal belongs.

Compared with the existing atrial fibrillation technology, in this embodiment of this application, whether an electrocardiosignal is an atrial fibrillation signal is not determined when an atrial fibrillation event occurs, but a signal type of a non-atrial fibrillation electrocardiosignal may be further marked when a non-atrial fibrillation event occurs, so that an atrial fibrillation event can be predicted for the user. In this way, the user can be notified in advance, an atrial fibrillation event occurrence of the user that is not prewarned can be prevented, and a detection effect and a detection range are improved.

It should be noted that the atrial fibrillation signal classification model may also be used to identify an atrial fibrillation signal, that is, a corresponding electrocardiosignal generated when the user encounters an atrial fibrillation event. In this case, a signal type corresponding to the electrocardiosignal generated when the atrial fibrillation event occurs is an atrial fibrillation signal type.

In a possible implementation, different types of standard signals may be set in the atrial fibrillation signal classification model. In this case, the terminal device may directly use the electrocardiosignal as an input parameter of the model, and the atrial fibrillation signal classification model may perform matching between the input electrocardiosignal and the standard signals of various types, and identify the signal type of the electrocardiosignal based on a matching result. By way of example but not limitation, the terminal device may calculate and identify a matching degree between each standard signal and the electrocardiosignal, and select a signal type of a standard signal with a largest matching degree value as the signal type of the electrocardiosignal. If there are two or more standard signals whose values of matching degrees with the electrocardiosignal are the same and the largest, the electrocardiosignal may be divided into a plurality of signal segments by using a preset signal segmentation algorithm, a matching degree between each signal segment and the plurality of standard signals of candidate types with the highest matching degree is calculated, a subtype associated with each signal segment is identified, and the signal type of the entire electrocardiosignal is identified based on subtypes of all the signal segments.

For example, the terminal device identifies that a value of a matching degree between the electrocardiosignal and a standard signal of a first type and a value of a matching degree between the electrocardiosignal and a standard signal of a second type are the same and the largest. In this case, the terminal device may divide the electrocardiosignal into three signal segments, which are a first signal segment, a second signal segment, and a third signal segment, calculate matching degrees between the foregoing three signal segments and the standard signals of the foregoing two types, and determine a subtype corresponding to each signal segment. Identification results are: the first signal segment (the first type), the second signal segment (the first type), and the third signal segment (the second type). Therefore, it may be determined that a quantity of signal segments that match the first type is greater than a quantity of signal segments that match the second type. It is determined that the signal type of the electrocardiosignal is the first type.

Optionally, before calculating the matching degree between the electrocardiosignal and the standard signal, the terminal device may perform standardization processing on the electrocardiosignal. The terminal device adjusts signal duration of the electrocardiosignal based on standard duration of the standard signal, so that the standard duration is the same as the adjusted signal duration. If the signal duration of the electrocardiosignal is greater than the standard duration, the electrocardiosignal may be cropped, and signal duration of an electrocardiosignal obtained through cropping is the same as the signal duration of the standard signal. If the signal duration of the electrocardiosignal is less than the standard duration, a lacking area may be filled through signal stretching, cyclic extension, or the like, so that the signal duration of the adjusted electrocardiosignal is the same as the standard duration of the standard signal. After standardization processing is performed, the matching degree between the signals may be calculated, and the signal type of the electrocardiosignal is identified based on the matching degree.

Optionally, when the matching degree between the electrocardiosignal and the standard signal is calculated, a dynamic time warping algorithm may be used. An implementation manner is specifically as follows. The terminal device may convert the electrocardiosignal into a heart rate sequence based on a heart rate value collected at each collect time. A corresponding coordinate grid is generated based on the heart rate sequence and a quantity of elements included in a standard sequence corresponding to the standard signal. A difference between elements corresponding to intersection points of the coordinate grid is used as an element distance value corresponding to the coordinate grid. When a total distance of a path is calculated, element distance values corresponding to the intersection points of the grid on the path are superimposed, to obtain a total distance value corresponding to the path. A path with a minimum total distance value is selected as a feature path between the heart rate sequence and the standard sequence, and the distance value corresponding to the feature path is used as a distance value between the electrocardiosignal and the standard signal. A matching degree between the two signals is calculated based on the distance value.

For example, the electrocardiosignal fed back by the target user has M collect moments. A heart rate sequence including M elements is generated based on a heart rate value corresponding to each collect moment. The standard sequence corresponding to the standard signal includes N elements. An M x N coordinate grid may be generated based on the foregoing two sequences. A coordinate distance value of coordinates (m, n) is a distance value between the $m^{th}$ element in the heart rate sequence and the $n^{th}$ element in the standard sequence. A path with a minimum total distance value in all paths to a target point (M, N) is calculated and used as a feature path, and the total distance value corresponding to the feature path is used as the distance value between the electrocardiosignal and the standard signal.

In a possible implementation, the terminal device may calculate feature parameters of the electrocardiosignal in a plurality of feature dimensions, convert the electrocardiosignal into a signal feature sequence, use the signal feature sequence as an input of the atrial fibrillation signal classification model, and output the signal type of the electrocardiosignal. Optionally, a feature extraction layer may be further set in the atrial fibrillation signal classification model. The terminal device inputs the electrocardiosignal into the atrial fibrillation signal classification model, converts the electrocardiosignal into the feature sequence by using the feature extraction layer in the model, sends the feature sequence to a signal classification layer connected to the feature extraction layer, calculates a matching degree between the feature sequence and each candidate type, uses the matching degree as a probability value corresponding to the candidate type, imports all probability values into a fully-connected layer, and outputs the signal type of the electrocardiosignal. A signal feature of the electrocardiosignal includes but is not limited to one or a combination of the following: a maximum heart rate value, a minimum heart rate value, first heart rate duration (that is, duration exceeding a first heart rate threshold), second heart rate duration (that is, duration less than a second heart rate threshold), and the like. Optionally, in addition to determining a signal feature value based on an electrocardiosignal currently fed back, the terminal device may further determine a joint feature value, for example, a change rate of an average heart rate, based on the electrocardiosignal and an associated signal whose collect time is adjacent to that of the electrocardiosignal, and form the foregoing signal feature sequence by using the signal feature value determined based on the single signal and the joint feature value.

S403. Calculate, based on the signal type of the electrocardiosignal, a risk level of an atrial fibrillation occurrence, to predict whether the target user is to have an atrial fibrillation attack.

In this embodiment, after identifying the signal type of the electrocardiosignal, the terminal device may predict, based on signal types of a plurality of electrocardiosignals of the target user, a probability value of the occurrence of the atrial fibrillation event of the target user, that is, the atrial fibrillation occurrence probability. Before the atrial fibrillation event occurs, a vital sign of a human body shows a specific precursor, that is, electrocardiosignals closer to the atrial fibrillation event have specific commonality. By determining the signal type of the electrocardiosignal, it may be determined whether the electrocardiosignal meets the common characteristic of the electrocardiosignal close to the atrial fibrillation event. Therefore, the occurrence probability of the atrial fibrillation event of the user may be determined based on the signal type of the electrocardiosignal. However, it may be determined, based on the signal types of the plurality of electrocardiosignals, whether the commonality between the electrocardiosignal of the target user and an electrocardiosignal generated before the atrial fibrillation event occurrence occurs occasionally or frequently. In addition, the atrial fibrillation occurrence probability of the target user can be accurately determined based on a determining result, so that the atrial fibrillation event of the user can be predicted, and a situation in which the user is warned only when the atrial fibrillation event occurs is avoided.

In a possible implementation, the terminal device may sequentially combine the signal types of the electrocardiosignals based on an order of collect times of the electrocardiosignals, to generate a signal type sequence. The signal type sequence is imported into a probability conversion function, and the atrial fibrillation occurrence probability of the target user is calculated.

In a possible implementation, the terminal device may generate a corresponding probability value based on a signal type of each electrocardiosignal, perform weighted superimposition on a plurality of probability values, and calculate the atrial fibrillation occurrence probability of the target user. A weighted value of each electrocardiosignal is determined based on a difference between a collect time and a current time. A smaller difference between the collect time and the current time indicates a larger corresponding weight. On the contrary, a larger difference between the collect time and the current time indicates a smaller corresponding weight. Optionally, an algorithm for calculating the atrial fibrillation occurrence probability may be specifically:

$$Probability = \sum_{i=1}^{N} \frac{SignalType_i}{CurrentTime - CollectTime_i},$$

where

*Probability* is the atrial fibrillation occurrence probability; *SignalType_i* is a probability value corresponding to a signal type of the ith electrocardiosignal; *CurrentTime* is the current time; *CollectTime_i* is a collect time of the ith electrocardiosignal; and N is a total quantity of the obtained electrocardiosignals.

In this embodiment, the terminal device may output the atrial fibrillation occurrence probability to the user by using a built-in interaction module. An output manner includes but is not limited to: performing output by using a notification, performing output by broadcasting, or adding, to a preset interface, a component used to feed back the atrial fibrillation occurrence probability in real time, and performing output by adjusting a corresponding parameter value in the component.

In a possible implementation, the terminal device may obtain an explanatory paragraph and atrial fibrillation information that are associated with the atrial fibrillation occurrence probability, and generate an atrial fibrillation report by using the atrial fibrillation occurrence probability, the explanatory paragraph, and the atrial fibrillation information. The user may obtain, by reading the atrial fibrillation report, more information related to the atrial fibrillation trigger probability. This improves readability of the atrial fibrillation occurrence probability, and helps the user learn of a physical condition of the user. The terminal device may push corresponding atrial fibrillation information to the user from a cloud database based on a current atrial fibrillation occurrence probability of the user. In this way, a matching degree between the pushed information and the user is improved, and precise information push is achieved.

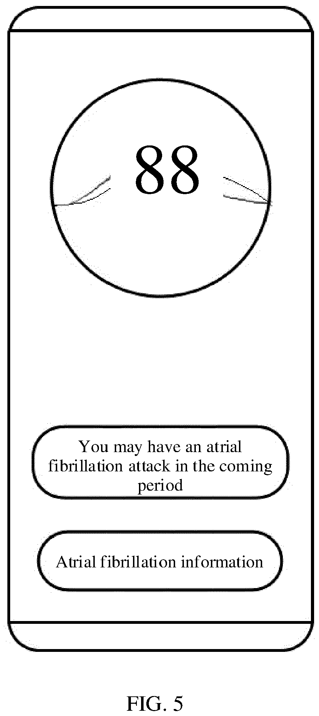

FIG. 5 is a schematic diagram of outputting an atrial fibrillation occurrence probability according to an embodiment of this application. Refer to FIG. 5. A terminal device may determine the atrial fibrillation occurrence probability of a target user based on signal types of a plurality of electrocardiosignals. A value displayed in the figure is 88, a corresponding explanatory paragraph is provided under the atrial fibrillation occurrence probability, which is "you may have an atrial fibrillation attack in the coming period", and atrial fibrillation information is added under the explanatory paragraph. A user may click a UI control corresponding to the atrial fibrillation information to read specific information.

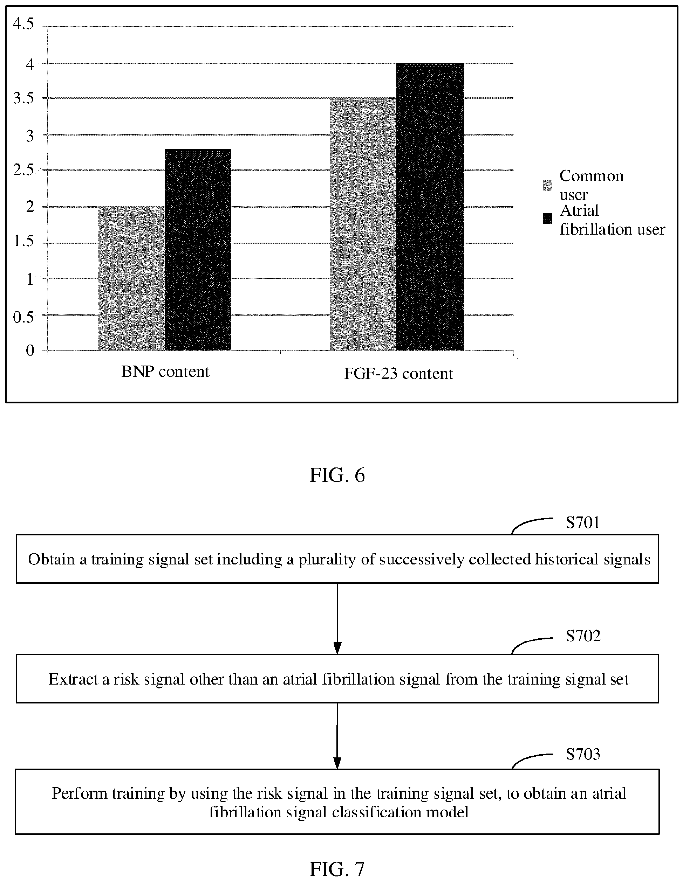

In a possible implementation, in the existing atrial fibrillation technology, the probability of the atrial fibrillation event occurrence of the user may be predicted by detecting content of a marker related to atrial fibrillation in a body of the user. For example, the probability of the atrial fibrillation event occurrence of the user may be determined by detecting content of BNP or FGF-23 of the user. FIG. 6 is a schematic diagram of comparison between related markers of an atrial fibrillation patient and a common patient according to an embodiment of this application. Refer to FIG. 6. Content of FGF-23 and BNP of the atrial fibrillation patient is higher than that of a common user. Whether the user is an atrial fibrillation patient may be determined by setting a corresponding content threshold, to predict whether an atrial fibrillation event of the user is to be triggered. However, because in addition to atrial fibrillation, the content of the marker related to atrial fibrillation is also related to other diseases, it cannot be directly determined that the user has an atrial fibrillation behavior based on the marker. In other words, identification accuracy in the foregoing manner is low.

In a possible implementation, in the existing atrial fibrillation technology, a corresponding risk scoring table may be configured based on a plurality of risk factors greatly associated with the atrial fibrillation event, a corresponding contribution value is configured for each risk factor, a risk item that the user has is determined by obtaining user information and comparing the user information with each risk factor, a total assessment score of the user is calculated based on contribution values corresponding to all risk items, and whether the user is an atrial fibrillation patient is determined based on the total assessment score. In this way, the atrial fibrillation event of the user is predicted. By way of example but not limitation, Table 1 shows an atrial fibrillation risk scoring table provided in an embodiment of this application. CHADS and CHA2DS2-VASc scoring methods are used in the atrial fibrillation risk scoring table and the following eight risk factors are included: congestive heart failure/left ventricular dysfunction, hypertension, aged 75 or above, diabetes, cerebral stroke/TIA/thromboembolism history, vascular disease, aged 65 to 74, gender (female). Each project has a corresponding contribution value. The terminal device may determine the score of the user by comparing a quantity of risk factors that match the user information with corresponding scores, to predict the probability of the atrial fibrillation event occurrence of the user. However, individual differences are not considered in the foregoing manner, which results in low scoring accuracy. In addition, it may be determined, based on the risk factors, that the scoring manner is mainly for the elderly and cannot cover people of all groups, and therefore the scoring manner has a small application scope.

**Table 1**

|  | CHADS | CHA2DS2-VASc |
|---|---|---|
| Congestive heart failure/left ventricular dysfunction | 1 | 1 |

(continued)

|  | CHADS | CHA2DS2-VASc |
|---|---|---|
| Hypertension | 1 | 1 |
| Aged 75 or above | 1 | 2 |
| Diabetes | 1 | 1 |
| Cerebral stroke/TIA/thromboembolism history | 2 | 2 |
| Vascular disease | / | 1 |
| Aged 65 to 74 | / | 1 |
| Gender (female) | / | 1 |
| Total score | 6 | 9 |

Different from that in the foregoing two technologies, in this embodiment of this application, the electrocardiosignal of the user can be collected in daily life, a signal type of each user's electrocardiosignal may be determined, and an atrial fibrillation occurrence probability of the user is calculated based on signal types of a plurality of electrocardiosignals. By observing the electrocardiosignal of the user for a period of time, the atrial fibrillation occurrence probability of the user can be accurately predicted, and prediction accuracy can be improved. In addition, electrocardiosignal collection is applicable to people of all groups, and therefore an application scope is expanded.

It can be learned from the foregoing descriptions that, according to the electrocardiosignal prediction method provided in this embodiment of this application, the signal type of each electrocardiosignal is determined by importing the collected electrocardiosignal into a preset atrial fibrillation signal classification model, the signal types of the electrocardiosignals of the user in the plurality of collect periods are obtained, the atrial fibrillation occurrence probability of the target user is calculated based on the signal types of the plurality of electrocardiosignals, and the atrial fibrillation event can be predicted based on a value of the atrial fibrillation occurrence probability. Therefore, it is convenient for the user to determine a physical condition of the user, and a detection effect and user experience are improved.

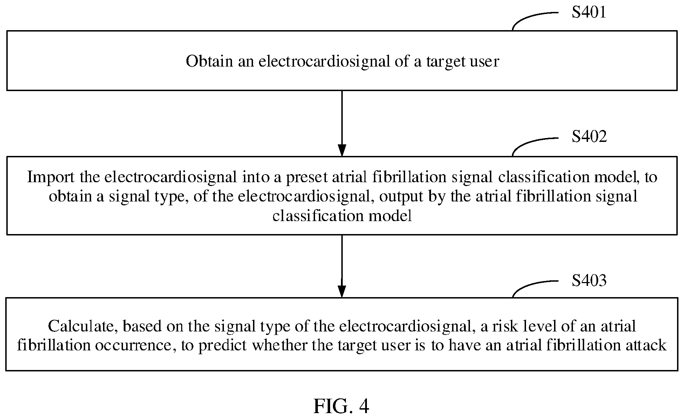

FIG. 7 is a specific implementation flowchart of an electrocardiosignal prediction method according to a second embodiment of this application. Refer to FIG. 7. Compared with the method in the embodiment in FIG. 4, according to the electrocardiosignal prediction method in this embodiment, before the importing the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample, the method further includes S701 to S703. Details are as follows.

S701. Obtain a training signal set including a plurality of successively collected historical signals, where the training signal set includes at least one atrial fibrillation signal.

In this embodiment, before identifying the electrocardiosignal, the terminal device may train a preset original training model by using the training signal set, to obtain an atrial fibrillation classification model that can be used to identify the signal type of the electrocardiosignal. Based on this, the terminal device needs to obtain a plurality of training signal sets. An obtaining manner may be as follows. If the terminal device is an intelligent device such as a smartphone or a tablet computer used by the user, the intelligent device may download the plurality of training signal sets from a cloud database by establishing a communication connection to the cloud database. If the terminal device is a server, the server may be configured to receive electrocardiosignals fed back by electronic devices, and store the electrocardiosignals in a local database. In this case, the server may directly extract the training signal sets from the local database. Each training signal set includes a plurality of historical signals, and an order of the historical signals in the signal set matches an order of collect times of the historical signals.

In a possible implementation, the training signal set is specifically a training signal set including electrocardiosignals of an atrial fibrillation patient. The atrial fibrillation signal classification model is specifically used to determine whether a signal feature of the electrocardiosignal has a precursor feature of the atrial fibrillation event occurrence. Therefore, during training of the atrial fibrillation signal classification model, a signal generated before the atrial fibrillation event occurs needs to be collected, and the precursor feature is determined in the signal generated before the atrial fibrillation event occurs, to predict the atrial fibrillation event. For the foregoing reason, when obtaining the training signal set, the terminal device needs to collect a signal including the atrial fibrillation event, that is, an atrial fibrillation signal, and a user having the atrial fibrillation signal is an atrial fibrillation patient. Based on this, the terminal device may select a target training user based on an atrial fibrillation patient identifier of each user, and obtain a signal set corresponding to each target training user as the foregoing training signal set. Each training signal set includes at least one atrial fibrillation signal.

In a possible implementation, if the terminal device is specifically an intelligent terminal of a user, S701 may be specifically: The terminal device may obtain user information of the target user, where the user information may be used to indicate a biometric feature of the user, which includes but is not limited to one or a combination of the following: an age of the user, a gender of the user, a current health status, a disease record, and the like. The terminal device may identify, from the cloud database, a training object that matches the user information, and obtain an electrocardiosignal set of the training object as the training signal set, so that an atrial fibrillation signal classification model that matches the user can be customized for the user. This improves accuracy of subsequent training.

In a possible implementation, if the terminal device is specifically a cloud server, that is, the terminal device is configured to respond to a signal type of an electrocardiosignal uploaded by each target user, S701 may be specifically: The terminal device may classify existing users in the database into a plurality of user groups according to a preset user group classification rule, and configure corresponding atrial fibrillation signal classification models for different user groups. Based on this, during training of the atrial fibrillation signal classification model, the atrial fibrillation signal classification model is trained by using a training signal set in a user group associated with the model. In this way, an objective of configuring different atrial fibrillation signal classification models for people with different features is achieved. In a response process, the terminal device obtains the user information of the target user, determines, based on the user information, a user group to which the target user belongs, and identifies the electrocardiosignal of the target user by using an atrial fibrillation signal classification model associated with the user group.

S702. Extract a risk signal other than the atrial fibrillation signal from the training signal set.

In this embodiment, the terminal device may perform an atrial fibrillation signal determining operation on each historical signal in the training signal set, identify a corresponding atrial fibrillation signal when the atrial fibrillation event occurs, mark the atrial fibrillation signal, and identify another non-atrial fibrillation historical signal other than the atrial fibrillation signal as the risk signal. An atrial fibrillation feature parameter may be configured for the terminal device. The terminal device may extract a signal feature value of each historical signal, perform matching between the signal feature value of each historical signal and the atrial fibrillation feature parameter, and determine, based on a matching result, whether the historical signal is an atrial fibrillation signal.

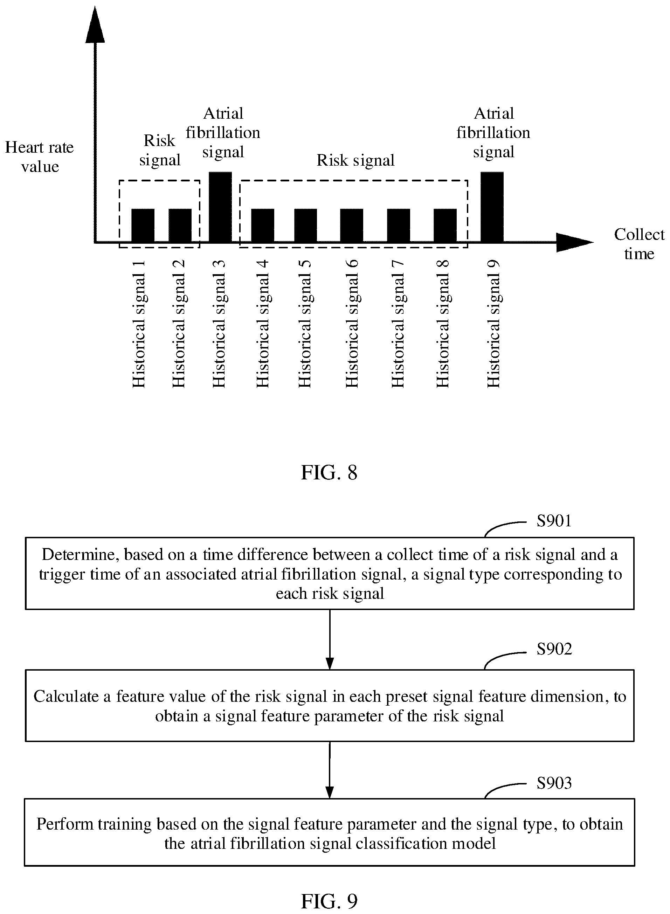

By way of example but not limitation, FIG. 8 is a schematic diagram of a training signal set according to an embodiment of this application. Refer to FIG. 8. The training signal set includes 10 historical signals, and each historical signal is identified by using a rectangular area, namely, a PPG column. A height of the PPG column may be determined based on an average heart rate value within a collect time. If the average heart rate value within the collect time is high, the PPG column is high. On the contrary, if the PPG column is short, it indicates that the average heart rate value corresponding to a historical signal within the collect time is low. An atrial fibrillation event means that an atrium beats very fast in a period of time, and sometimes the atrium beats more than 200 times per minute. In this case, an electrocardiosignal is characterized by a high average heart rate. Therefore, an electrocardiosignal that indicates an atrial fibrillation event, that is, the atrial fibrillation signal marked in the figure, may be identified based on the height of the PPG column. After obtaining the atrial fibrillation signal through identification, the terminal device may identify the historical signal other than the foregoing atrial fibrillation signal as the risk signal.

S703. Train a preset original classification model by using the risk signal in the training signal set, to obtain the atrial fibrillation signal classification model.

In this embodiment, the terminal device may perform training by using the obtained risk signal. The terminal device may mark each risk signal with a corresponding signal type in advance, and construct a training sample based on the mark information and the risk signal. The terminal device imports a plurality of risk signals into the original classification model, obtains a plurality of predicted types through calculation, identifies whether each predicted type matches a preset signal type of a risk signal corresponding to the predicted type, to obtain, through calculation, a predicted loss value corresponding to the original classification model, and determines whether the original classification model converges and whether the predicted loss value is less than a preset loss threshold. If yes, it is identified that adjustment to the original classification model is completed, and the adjusted original classification model is identified as the atrial fibrillation signal classification model. On the contrary, if the original classification model does not converge, a learning parameter in the original classification model needs to be adjusted, so that the foregoing original classification model meets the two conditions of convergence and the predicted loss value being less than the preset loss threshold.

In a possible implementation, the terminal device may be configured with a plurality of original classification models of different types, simultaneously perform training and learning of the plurality of original classification models by using the risk signals, select a preferred original classification model based on the two types of parameters, namely, convergence times and loss values corresponding to the plurality of original classification models, and construct the atrial fibrillation signal classification model based on the preferred original classification model.

In a possible implementation, based on a collect time difference between a risk signal and an atrial fibrillation signal associated with the risk signal, the terminal device may determine a training weight of the risk signal. A smaller collect time difference indicates a larger corresponding training weight. The atrial fibrillation signal associated with the risk signal is specifically an atrial fibrillation signal that is in all atrial fibrillation signals whose collect times are after a collect time of the risk signal and that has a minimum collect time difference with the risk signal, that is, an atrial fibrillation signal closest to the risk signal. Refer to FIG. 8. For a historical signal 1 and a historical signal 2, an atrial fibrillation signal associated with the historical signal 1 and the historical signal 2 is a historical signal 3. For a historical signal 4, because a collect time of the historical signal 3 is earlier than that of the historical signal 4, even though a collect time difference between the historical signal 3 and the historical signal 4 is the smallest, the historical signal 3 is not considered as an associated atrial fibrillation signal of the historical signal 4, in other words, the atrial fibrillation signal associated with the historical signal 4 is a historical signal 9. Training and learning based on the training weight of each risk signal can improve a training contribution of a risk signal close to an occurrence time of the atrial fibrillation event. A closer collect time indicates that more precursor features of the atrial fibrillation event are included and prediction is more accurate. On the contrary, a farther collect time indicates that fewer precursor features are included.

In this embodiment of this application, the training signal set is obtained, the historical signals in the training signal set are filtered, the risk signal is extracted, and training and learning are performed, to obtain the atrial fibrillation signal classification model. In this way, the atrial fibrillation event can be predicted, and prediction accuracy is improved.

FIG. 9 is a specific implementation flowchart of S703 in an electrocardiosignal prediction method according to a third embodiment of this application. Refer to FIG. 9. Compared with the step in the embodiment in FIG. 7, in the electrocardiosignal prediction method provided in this embodiment, S703 includes S901 to S903. Details are as follows.

Further, the performing training on a preset original classification model by using the risk signal in the training signal set, to obtain the atrial fibrillation signal classification model includes.

S901. Determine, based on a time difference between a collect time of the risk signal and a trigger time of an associated atrial fibrillation signal, a signal type corresponding to each risk signal.

In this embodiment, the terminal device may identify a collect time corresponding to each risk signal, and select, from the training signal set based on the collect time, the atrial fibrillation signal associated with the risk signal. The associated atrial fibrillation signal may be specifically the atrial fibrillation signal that is in all the atrial fibrillation signals whose collect times are after the collect time of the risk signal and that has the minimum collect time difference with the risk signal, that is, the atrial fibrillation signal closest to the risk signal. The terminal device may configure corresponding time difference ranges for different signal types, and identify, based on a time difference range which a time difference between a collect time of a risk signal and a trigger time of an associated atrial fibrillation signal is in, a signal type corresponding to the risk signal.

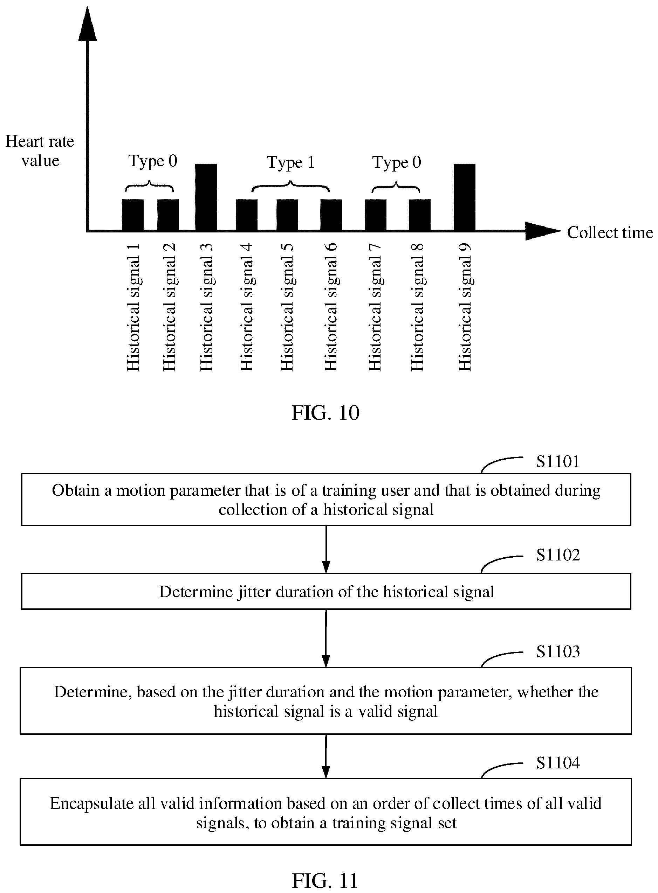

By way of example but not limitation, the terminal device may classify the signal types into two types: type 0 signal and type 1 signal. A collect time difference between the type 0 signal and the associated atrial fibrillation signal is within a range of a preset time threshold, and a collect time difference between the type 1 signal and the associated atrial fibrillation signal is beyond the range of the preset time threshold. The time threshold may be two hours. For example, if a collect time of a risk signal is 15 o'clock, and a collect time of an associated atrial fibrillation signal is 16 o'clock, a collect time difference between the two signals is 1 hour, and it may be determined that the signal type of the risk signal is type 0. If a collect time of another risk signal is 10 o'clock, and a collect time of an associated atrial fibrillation signal is 16 o'clock, a time difference between the two collect times is 6 hours, and it may be determined that the signal type of the risk signal is type 1. FIG. 10 is a schematic diagram of a marker of a signal type according to an embodiment of this application. Refer to FIG. 10. A collect period of each historical signal is one hour, that is, a time difference between two adjacent atrial fibrillation signals is one hour. The time threshold is two hours. Then, it may be determined that time differences between the associated atrial fibrillation signal (namely, the historical signal 3) and each of the historical signal 1 and the historical signal 2 are within two hours. In this case, both the two risk signals are type 0 signals. Time differences between the associated atrial fibrillation signal (namely, the historical signal 9) and each of the historical signal 4, the historical signal 5, and the historical signal 6 are all greater than two hours. In this case, the three risk signals are type 1 signals. By analogy, the signal type of each risk signal can be determined.

Certainly, the terminal device may alternatively classify the atrial fibrillation types into N types instead of two types, and configure corresponding time thresholds for different signal types. For example, a collect time difference between a signal of a first type and an associated atrial fibrillation signal is between t0 and t1, a collect time difference between a signal of a second type and an associated atrial fibrillation signal is between t1 and t2, ..., and a collect time difference between a signal of an Nth type and an associated atrial fibrillation signal is between tN-1 and tN. In this case, after the terminal device generates the atrial fibrillation signal classification model trained in the foregoing manner, and imports the actually collected electrocardiosignal into the foregoing model to determine the signal type of the risk signal, the terminal device may determine a predicted time period during which a next atrial fibrillation event occurs based on the signal type of the electrocardiosignal in addition to determining the probability of the atrial fibrillation event occurrence. Because the time difference between the foregoing signal type and an atrial fibrillation signal occurrence event is in a one-to-one correspondence, each signal type may correspond to one prediction time. The terminal device may query, based on the signal type of the electrocardiosignal, a prediction time associated with the electrocardiosignal, and prompt the user based on the prediction time.

S902. Calculate a feature value of the risk signal in each preset signal feature dimension, to obtain a signal feature parameter of the risk signal.

In this embodiment, a plurality of signal feature dimensions may be configured for the terminal device, and different signal feature dimensions are used to represent different signal features of the electrocardiosignal. The terminal device may parse the risk signal, determine the feature value of the risk signal in each signal feature dimension, and import each feature value into a parameter template based on a corresponding location of each signal feature dimension in the parameter template, to generate the signal feature parameter of the risk signal. The signal feature dimension includes but is not limited to one or a combination of the following: an average heart rate, a maximum heart rate, a minimum heart rate, duration in which a heart rate exceeds a first heart rate threshold, duration in which a heart rate is less than a second heart rate threshold, and the like.

S903. Perform training based on the signal feature parameter and the signal type, to obtain the atrial fibrillation signal classification model.

In this embodiment, after establishing the signal feature parameter and the signal type of each risk signal, the terminal device generates a training sample, performs training by using a plurality of training samples, adjusts the parameter in the original classification model until a result converges, and identifies the adjusted original classification model as the atrial fibrillation signal classification model.

In this embodiment of this application, risk type marking is performed based on the time difference between the risk signal and the associated atrial fibrillation signal, so that the risk signal having the precursor feature of the atrial fibrillation event can be extracted, and the atrial fibrillation event can be predicted based on the signal type of the electrocardiosignal obtained through identification. In this way, a detection effect is improved.

FIG. 11 is a specific implementation flowchart of S701 in an electrocardiosignal prediction method according to a fourth embodiment of this application. Refer to FIG. 11. Compared with the step in the embodiment in FIG. 7, in the electrocardiosignal prediction method provided in this embodiment, S701 includes S1101 to S1104. Details are as follows.

Further, the obtaining a training signal set including a plurality of successively collected electrocardiosignals includes the following steps.

S1101. Obtain a motion parameter that is of the training user and that is obtained during collection of the historical signal.

In this embodiment, before importing the historical signals of the training signal set into the original classification model for training, the terminal device may perform filtering on the historical signals in the training signal set, to filter out a historical signal with a poor collection quality, so that accuracy of subsequent training can be improved. Based on this, when obtaining the electrocardiosignal of the user, in addition to feeding back the electrocardiosignal waveform, the wearable device may further add an associated motion parameter to the electrocardiosignal, and feed back the foregoing two pieces of data to the terminal device. The terminal device may determine, based on a motion parameter associated with each historical signal, a motion status of the training user during collection of the historical signals of the training user. When the user performs intense exercise, a heart rate value increases. However, the increase is not caused by an atrial fibrillation event, and is a normal response to the exercise. In addition, during the exercise, the wearable device is not in close contact with skin of the user, and swaying and the like may occur, which affects a collection quality of the electrocardiosignal. Based on this, the terminal device may determine validity of the historical signal based on the foregoing motion parameter.

The motion parameter may be obtained by using a motion sensing module such as an acceleration sensor and a gyroscope in the wearable device. The terminal device may determine a motion status of a historical user based on a sensing value fed back by the motion sensing module.

S1102. Determine jitter duration of the historical signal.

In this embodiment, the terminal device may parse a signal waveform of the historical signal, determines a waveform segment in which jitter occurs, and use duration of the waveform segment as jitter duration of the historical signal. The jitter waveform segment may be a waveform segment of an interruption area in the collection process, or may be a waveform segment whose waveform change frequency is higher than a normal value.

S1103. Determine, based on the jitter duration and the motion parameter, whether the historical signal is a valid signal.

In this embodiment, the terminal device may calculate signal quality of the historical signal by using the foregoing two parameters, and compare the calculated signal quality with a preset quality threshold, to determine whether the historical signal is a valid signal. If the signal quality is greater than or equal to the quality threshold, the historical signal is identified as the valid signal. On the contrary, if the signal quality is less than the quality threshold, the historical signal is identified as an invalid signal.

In a possible implementation, a manner of calculating the signal quality may be: determining a first quality factor based on a ratio of the jitter duration to signal duration of the historical signal, where longer jitter duration indicates a smaller value of the first quality factor; determining a second quality factor based on a ratio of the motion parameter to a static motion parameter, where a larger value of the motion parameter indicates a larger motion amplitude of the user and a smaller value of the corresponding second quality factor; and performing weighted summation on the foregoing two quality factors to calculate the signal quality of the historical signal.

S1104. Encapsulate all valid information based on an order of collect times of all valid signals, to obtain the training signal set.

In this embodiment, the terminal device determines, based on the order of the collect times of all valid signals, a signal order of each valid signal in the training signal set, to encapsulate the plurality of valid signals, filter out an invalid signal, and form the foregoing training signal set. In this way, accuracy of a subsequent training process is improved.

In this embodiment of this application, filtering is performed on the historical signals, to filter out the invalid signal, so that accuracy of the subsequent training operation can be improved.

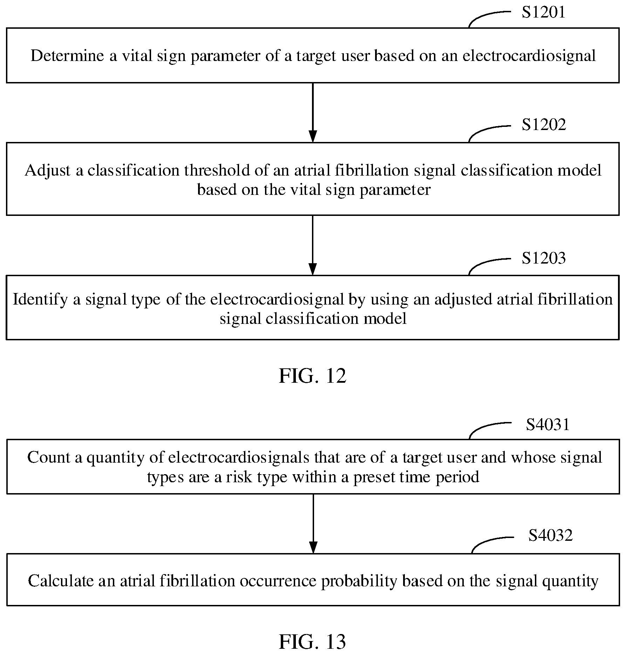

FIG. 12 is a specific implementation flowchart of S402 in an electrocardiosignal prediction method according to a fifth embodiment of this application. Refer to FIG. 12. Compared with the step in any one of the embodiments in FIG. 4, FIG. 7, FIG. 9, and FIG. 11, in the electrocardiosignal prediction method provided in this embodiment, S402 includes S1201 to S1203. Details are as follows.

Further, the importing the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample includes the following steps.

S1201. Determine a vital sign parameter of the target user based on the electrocardiosignal.

In this embodiment, before performing classification on the electrocardiosignal of the target user by using the atrial fibrillation signal classification model, the terminal device may adjust the atrial fibrillation signal classification model based on the vital sign of the user, which is a model warm-up phase, so that a subsequent signal type identification process is accurate.

In this embodiment, the terminal device may obtain the vital sign parameter of the target user through user input, wearable device feedback, and the like. For example, the vital sign parameter may be a resting heart rate of the target user, for example, a heart rate value corresponding to a status in which the user does not move for a long time (including a siting process and a sleep process), or a heart rate value in a moving process. By obtaining heart rate values in different statuses, a heart rate change amplitude and a heart rate reference value of the target user may be determined, and the foregoing values are used as vital sign parameters of the target user.

S1202. Adjust a classification threshold of the atrial fibrillation signal classification model based on the vital sign parameter.

In this embodiment, the terminal device may adjust the classification threshold in the atrial fibrillation signal classification model based on the collected vital sign parameter of the target user, so that a classification process can match a physical status of the target user. In this way, an objective of customizing a classification model for an individual can be achieved. For example, the terminal device may determine a heart rate change amplitude value of the target user based on a difference between a resting heart rate and a moving heart rate in the vital sign parameters, and adjust an atrial fibrillation trigger threshold based on the heart rate change amplitude value, to accurately determine whether a current heart rate value of the user approximates to the foregoing atrial fibrillation trigger threshold, and determine the atrial fibrillation occurrence probability corresponding to the electrocardiosignal.

SI203. Identify the signal type of the electrocardiosignal by using an adjusted atrial fibrillation signal classification model.

In this embodiment, after adjusting the classification threshold of the atrial fibrillation signal classification model based on the vital sign parameter of the target user, the terminal device may perform a signal type identification operation on the electrocardiosignal of the target user. For a specific description of the identification operation, reference may be made to the related descriptions in the foregoing embodiments, and details are not described herein again.

In this embodiment of this application, the vital sign parameter of the target user is obtained, and the atrial fibrillation signal classification model is adjusted based on the vital sign parameter, so that the atrial fibrillation signal classification model matches the target user, which improves accuracy of identifying an atrial fibrillation signal.

FIG. 13 is a specific implementation flowchart of S403 in an electrocardiosignal prediction method according to a sixth embodiment of this application. Refer to FIG. 13. Compared with the step in any one of the embodiments in FIG. 4, FIG. 7, FIG. 9, and FIG. 11, in the electrocardiosignal prediction method provided in this embodiment, S403 includes S4031 and S4032. Details are as follows.

Further, the calculating, based on the signal type of the electrocardiosignal, a risk level of an atrial fibrillation occurrence, to predict whether the target user is to have an atrial fibrillation attack includes the following steps.

S4031. Count a quantity of electrocardiosignals that are of the target user and whose signal types are a risk type within a preset time period.

In this embodiment, signal types preset by the terminal device include at least two types: the risk type and a non-risk type. A signal feature of an electrocardiosignal of the risk type includes a large quantity of precursor features of the atrial fibrillation event occurrence. In this case, the atrial fibrillation signal classification model may identify the electrocardiosignal of the foregoing type as the electrocardiosignal of the risk type. On the contrary, if a signal feature of an electrocardiosignal does not include the precursor features of the atrial fibrillation event occurrence or matches a small quantity of the precursor features of the atrial fibrillation event occurrence, the atrial fibrillation signal classification model may identify the electrocardiosignal of the foregoing type as an electrocardiosignal of the non-risk type. Certainly, according to different quantities of identifiable types of the atrial fibrillation classification model, there may be a plurality of cascaded subtypes under the risk type, for example, a first-level risk type and a second-level risk type. There may be a plurality of cascaded subtypes under the non-risk type. A specific quantity of cascaded types is determined based on a specific atrial fibrillation signal classification model, and is not limited herein.

By way of example but not limitation, as shown in the example in the foregoing embodiment, in the training process, the N signal types may be classified by the terminal device based on the time difference between the risk signal and the associated atrial fibrillation signal. The collect time of the signal of the first signal type is the closest to the trigger time of the atrial fibrillation event, and therefore the first signal type may be identified as the risk type. The collect times of the signals of the second signal type to the $N^{th}$ signal type are farther away from the trigger time of the atrial fibrillation event, and therefore the second signal type to the $N^{th}$ signal type may be identified as the non-risk type.

In this embodiment, the terminal device may obtain signal types of all electrocardiosignals collected within a preset time period, and count a quantity of signals whose signal types are the risk type. By way of example but not limitation, the preset time period may be one day or one week. After receiving a new electrocardiosignal, the terminal device counts a quantity of electrocardiosignals whose signal types are the risk type in all electrocardiosignals whose collect time differences between the new electrocardiosignal are within a preset time period, that is, the foregoing signal quantity. For example, a collect time of an electrocardiosignal is 18:00 on December 13, and the preset time period is one day. All electrocardiosignals collected between 18:00 on December 12 and 18:00 on December 13 are obtained, and a quantity of signals whose signal types are the risk type in the obtained electrocardiosignals is counted.

S4032. Calculate the atrial fibrillation occurrence probability based on the signal quantity.

In this embodiment, the terminal device may set an atrial fibrillation occurrence probability conversion function based on the quantity of electrocardiosignals of the risk type. The terminal device imports the foregoing signal quantity into the conversion function, and calculates the atrial fibrillation occurrence probability of the target user corresponding to a moment at which the electrocardiosignal is collected.

In a possible implementation, the terminal device may calculate an occurrence frequency of the risk signal of the target user based on the foregoing signal quantity and the preset time period, and determine the atrial fibrillation occurrence probability based on the occurrence frequency.

In a possible implementation, the terminal device may divide the time period into a plurality of sub-time periods, and collect statistics about occurrence frequencies of corresponding risk signals in the sub-time periods, to generate an occurrence frequency change curve of the risk signal corresponding to the time period, and determine the atrial fibrillation occurrence probability based on the occurrence change curve.

In this embodiment of this application, the quantity of electrocardiosignals of the risk type within the preset time period is counted, and the atrial fibrillation occurrence probability is determined based on the signal quantity, so that the atrial fibrillation event can be predicted based on the signal type of the electrocardiosignal. This improves the detection effect.

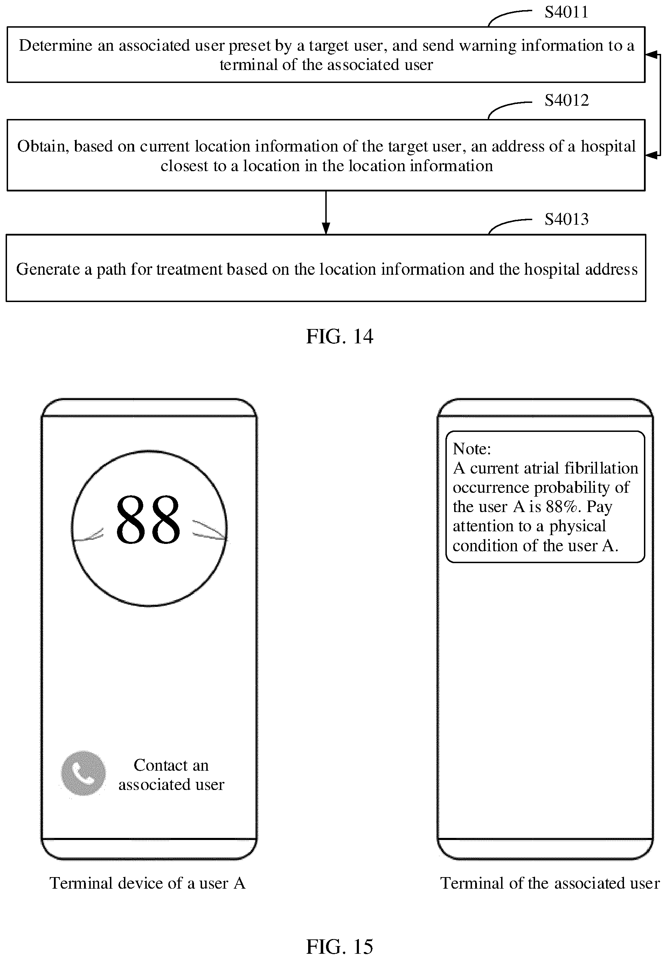

FIG. 14 is a specific implementation flowchart of an electrocardiosignal prediction method according to a seventh embodiment of this application. Refer to FIG. 14. Compared with the method in any one of the embodiments in FIG. 4, FIG. 7, FIG. 9, and FIG. 11, after the calculating, based on the signal type of the electrocardiosignal, a risk level of the atrial fibrillation occurrence, to predict whether the target user is to have an atrial fibrillation attack, the electrocardiosignal prediction method provided in this embodiment further includes S1401 to S1403. Details are as follows.

Further, if the atrial fibrillation occurrence probability is greater than a preset probability threshold, after the calculating, based on the signal type of the electrocardiosignal, a risk level of an atrial fibrillation occurrence, to predict whether the target user is to have an atrial fibrillation attack, the method further includes the following steps.

S1401. Determine an associated user preset by the target user, and send warning information to a terminal of the associated user.

In this embodiment, if it is detected that the atrial fibrillation occurrence probability of the target user is greater than the preset probability threshold, it indicates that the target user is very likely to encounter an atrial fibrillation event in the coming period. In this case, the target user needs to be warned. The warning manner may be notifying the user by using a pop-up window, prompt information, voice broadcast, an output prompt tone, or the like. In addition to the foregoing manner, the user may be notified in the manner described in S 1401 to S1403.

In this embodiment, the target user may preset the associated user and an address of the associated user. The terminal device sends the warning information to the terminal of the associated user according to the address that is of the associated user and that is preset by the target user, where the warning information may include the atrial fibrillation occurrence probability and an explanatory paragraph corresponding to the atrial fibrillation occurrence probability.

In a possible implementation, the terminal device may mark a shortcut call button of the associated user on a display interface. The target user may directly initiate a call request to the terminal of the associated user by using the shortcut call button of a phone. In this case, a contact number of the associated user may be a contact number of a guardian, a family member, or a related hospital of the target user. For example, FIG. 15 is a schematic diagram of outputting warning information according to an embodiment of this application. Refer to FIG. 15. The atrial fibrillation occurrence probability of the target user is 88%, and the preset probability threshold is 80%. In this case, the terminal device sends the warning information to the terminal of the associated user. Content of the warning information may be "A current atrial fibrillation occurrence probability of a user A is 88%. Pay attention to a physical condition of the user A." In addition, the shortcut call button for contacting the associated user may be set on the preset display interface, so that the user quickly notifies the associated user.

S 1402. Obtain, based on current location information of the target user, an address of a hospital closest to a location in the location information.

In this embodiment, when detecting that the atrial fibrillation occurrence probability of the target user is greater than the preset probability threshold, the terminal device may provide treatment information for the target user, which reduces operations for the user and improves information obtaining efficiency. In this case, the terminal device may obtain the current location information of the target user. If the terminal device is a smart device used by the user, the terminal device may obtain a positioning signal by using a built-in positioning module, and determine the location information of the target user based on the positioning signal. If the terminal device is a server, the terminal device may send a location obtaining request to a user terminal of the target user, and the user terminal may obtain the location information by using the built-in positioning module and feed back the location information to the server.

In this embodiment, the terminal device may mark the location information of the target user on a preset map interface. In a possible implementation, the terminal device may access a third-party server by using an application programming interface API, mark the location information on the preset map interface by using a map application program provided by the third-party server, and search for a hospital closest to the location of the user based on the location information, to determine an address of the hospital.

S1403. Generate a path for treatment based on the location information and the hospital address.

In this embodiment, after obtaining the current location information of the target user and the hospital address, the terminal device may generate the path for treatment starting from the location of the user and ending at the hospital. The terminal device may invoke the third-party map application, import the foregoing two locations into the third-party map application, and output the foregoing path for treatment by using a path generation algorithm of the third-party map application.

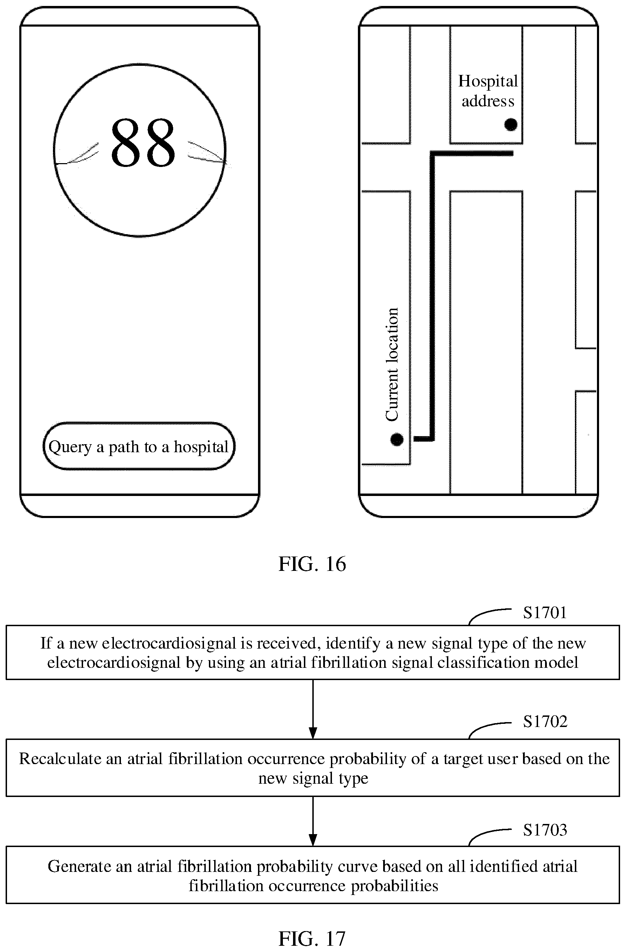

For example, FIG. 16 is a schematic diagram of outputting a path for treatment according to an embodiment of this application. Refer to FIG. 16. The terminal device may add a prompt button of "query a path to hospital" on the display interface. The terminal device may switch to a corresponding display page of the path for treatment when a click is performed on the prompt button. When the user performs the click operation, the terminal device may obtain the current location information of the user, determine the address of the hospital based on the location information, and generate the corresponding path for treatment.

In this embodiment of this application, when an atrial fibrillation occurrence probability of the target user is greater than the probability threshold, the warning information is sent to the associated user and the path for treatment is provided, to reduce operations that the target user needs to perform. This improves operation efficiency, and an objective of automatic release of the warning information is achieved.

FIG. 17 is a specific implementation flowchart of an electrocardiosignal prediction method according to an eighth embodiment of this application. Refer to FIG. 17. Compared with the method in any one of the embodiments in FIG. 4, FIG. 7, FIG. 9, and FIG. 11, the electrocardiosignal prediction method provided in this embodiment further includes S1701 to S1703. Details are as follows.

Further, after the identifying an atrial fibrillation type corresponding to each atrial fibrillation cluster, the method further includes the following steps.

S1701. If a new electrocardiosignal is received, identify a new signal type of the new electrocardiosignal by using the atrial fibrillation signal classification model.

In this embodiment, during use of the wearable device by the user, the wearable device may send a collected new electrocardiosignal to the terminal device at a preset feedback period. Each time the terminal device receives a new electrocardiosignal, the terminal device may determine a corresponding new signal type by using the foregoing trained atrial fibrillation signal classification model. A manner of determining the new signal type is described in the foregoing embodiment, and an implementation process is completely the same. Details are not described herein again.

S1702. Recalculate an atrial fibrillation occurrence probability of the target user based on the new signal type.

In this embodiment, the terminal device may recalculate the atrial fibrillation occurrence probability of the target user based on a previously identified atrial fibrillation occurrence probability and the new signal type of the new collected electrocardiosignal, and calculate the foregoing updated atrial fibrillation probability. A specific calculation manner may be as follows. The terminal device re-determines the atrial fibrillation occurrence probability of the target user by using a preset atrial fibrillation occurrence probability conversion algorithm based on signal types of all electrocardiosignals corresponding to the previous atrial fibrillation occurrence probability obtained through calculation and the new signal type of the currently collected new electrocardiosignal, to obtain the foregoing updated atrial fibrillation probability.

S1703. Generate an atrial fibrillation probability curve based on all identified atrial fibrillation occurrence probabilities.

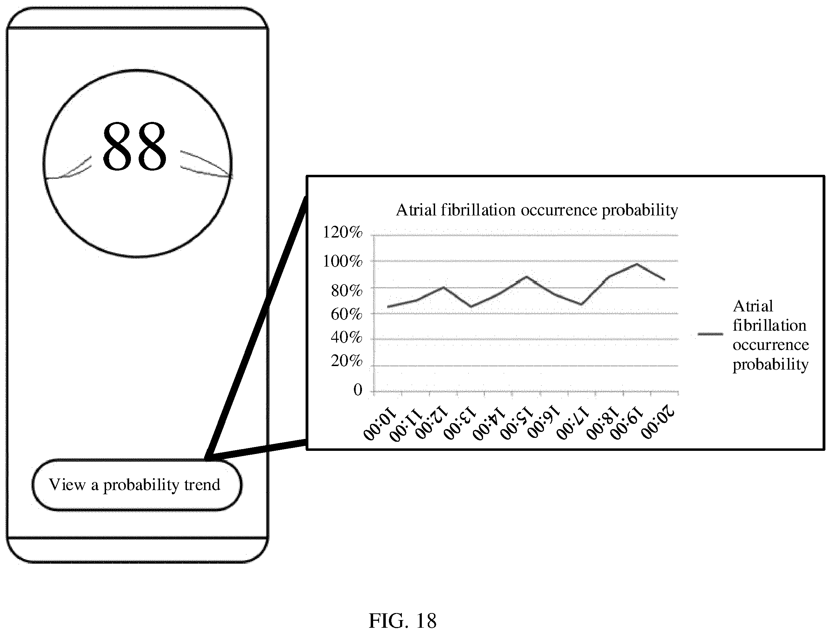

In this embodiment, each time after obtaining an atrial fibrillation occurrence probability of the target user through calculation, the terminal device may record the atrial fibrillation occurrence probability, to obtain a historical probability for each identification operation and an updated atrial fibrillation probability that is currently obtained through calculation, and the terminal device may generate the atrial fibrillation probability curve. This helps the target user determine a change trend of the atrial fibrillation occurrence probabilities. For example, FIG. 18 is a schematic diagram of outputting an atrial fibrillation probability curve according to an embodiment of this application. Refer to FIG. 18. Each time after obtaining the atrial fibrillation occurrence probability of the user through calculation, the terminal device may output a corresponding display interface, and the user may determine a current atrial fibrillation occurrence probability on the display interface. After collecting a new electrocardiosignal, the wearable device of the target user may send the electrocardiosignal to the terminal device, and the terminal device may identify a signal type of the new electrocardiosignal, and recalculate an atrial fibrillation occurrence probability of the target user based on the identified new signal type. When the user clicks a button "view a probability trend", a corresponding display page is displayed, the atrial fibrillation probability curve of the target user is output, and an atrial fibrillation occurrence probability corresponding to each update moment is determined.

In this embodiment of this application, each time an electrocardiosignal of the target user is received, the atrial fibrillation occurrence probability of the target user is recalculated, so that real-time performance of a warning operation can be improved, and a corresponding atrial fibrillation probability curve is output, so that the user determines a probability trend and learns of a physical condition of the user.

It should be understood that sequence numbers of the steps do not mean an execution sequence in the foregoing embodiments. The execution sequence of the processes should be determined based on functions and internal logic of the processes, and should not constitute any limitation on the implementation processes of embodiments of this application.

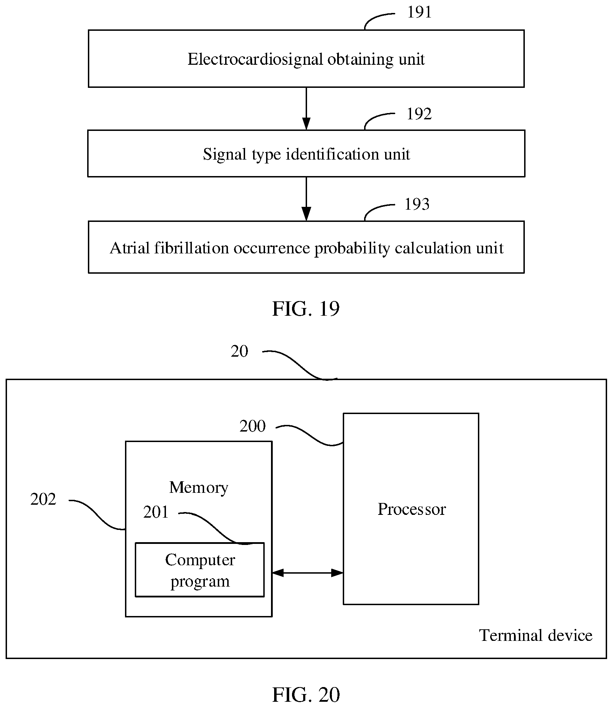

Corresponding to the electrocardiosignal prediction method in the foregoing embodiments, FIG. 19 is a block diagram of a structure of an electrocardiosignal prediction apparatus according to an embodiment of this application. For ease of description, only parts related to this embodiment of this application are shown.

Refer to FIG. 19. The electrocardiosignal prediction apparatus includes:

an electrocardiosignal obtaining unit 191, configured to obtain an electrocardiosignal of a target user;
a signal type identification unit 192, configured to import the electrocardiosignal into a preset atrial fibrillation signal classification model, to obtain a signal type, of the electrocardiosignal, output by the atrial fibrillation signal classification model, where the atrial fibrillation signal classification model is obtained through training with an atrial fibrillation patient being a model training sample; and
an atrial fibrillation occurrence probability calculation unit 193, configured to calculate, based on the signal type of the electrocardiosignal, a risk level of an atrial fibrillation occurrence, to predict whether the target user is to have an atrial fibrillation attack.

Optionally, the electrocardiosignal prediction apparatus further includes:

a training signal set obtaining unit, configured to obtain a training signal set including a plurality of successively collected historical signals, where the training signal set includes at least one atrial fibrillation signal;
a risk signal extraction unit, configured to extract a risk signal other than the atrial fibrillation signal from the training signal set; and
an atrial fibrillation signal classification model training unit, configured to perform training on a preset original classification model by using the risk signal in the training signal set, to obtain the atrial fibrillation signal classification model.

Optionally, the atrial fibrillation signal classification model training unit includes:

a signal type identification unit, configured to determine, based on a time difference between a collect time of the risk signal and a trigger time of an associated atrial fibrillation signal, a signal type corresponding to each risk signal;
a signal feature parameter generation unit, configured to calculate a feature value of the risk signal in each preset signal feature dimension, to obtain a signal feature parameter of the risk signal; and
a sample training unit, configured to perform training based on the signal feature parameter and the signal type, to obtain the atrial fibrillation signal classification model.

Optionally, the training signal set obtaining unit includes:

a motion parameter obtaining unit, configured to obtain a motion parameter that is of a training user and that is obtained during collection of the historical signal;

a jitter duration determining unit, configured to determine jitter duration of the historical signal;

a valid signal identification unit, configured to determine, based on the jitter duration and the motion parameter, whether the historical signal is a valid signal; and

a valid signal filter unit, configured to encapsulate all valid information based on an order of collect times of all valid signals, to obtain the training signal set.

Optionally, the signal type identification unit 192 includes:

a vital sign parameter obtaining unit, configured to determine a vital sign parameter of the target user based on the electrocardiosignal;

a classification threshold adjusting unit, configured to adjust a classification threshold of the atrial fibrillation signal classification model based on the vital sign parameter; and

an atrial fibrillation signal classification model invoking unit, configured to identify the signal type of the electrocardiosignal by using an adjusted atrial fibrillation signal classification model.

Optionally, the atrial fibrillation occurrence probability calculation unit 193 includes:

a signal quantity counting unit, configured to count a quantity of electrocardiosignals that are of the target user and whose signal types are a risk type within a preset time period; and

a signal quantity conversion unit, configured to calculate an atrial fibrillation occurrence probability based on the signal quantity.

Optionally, the electrocardiosignal prediction apparatus further includes:

a warning information sending unit, configured to: determine an associated user preset by the target user, and send warning information to a terminal of the associated user; and/or

a location information obtaining unit, configured to obtain, based on current location information of the target user, an address of a hospital closest to a location in the location information; and

a path for treatment generating unit, configured to generate a path for treatment based on the location information and the hospital address.

Optionally, the electrocardiosignal prediction apparatus further includes:

a new signal type determining unit, configured to: if a new electrocardiosignal is received, identify a new signal type of the new electrocardiosignal by using the atrial fibrillation signal classification model;

an atrial fibrillation occurrence probability updating unit, configured to recalculate an atrial fibrillation occurrence probability of the target user based on the new signal type; and

an atrial fibrillation probability curve generating unit, configured to generate an atrial fibrillation probability curve based on all identified atrial fibrillation occurrence probabilities.

In this way, the electrocardiosignal prediction apparatus provided in this embodiment of this application may determine the signal type of each electrocardiosignal by importing a collected electrocardiosignal into the preset atrial fibrillation signal classification model, obtain signal types of electrocardiosignals of the user in a plurality of collect periods, calculate the atrial fibrillation occurrence probability of the target user based on the signal types of the plurality of electrocardiosignals, and implement prediction of an atrial fibrillation event based on a value of the atrial fibrillation occurrence probability. Therefore, it is convenient for the user to determine a physical condition of the user, and a detection effect and user experience are improved.

FIG. 20 is a schematic diagram of a structure of a terminal device according to an embodiment of this application. As shown in FIG. 20, the terminal device 20 in this embodiment includes: at least one processor 200 (only one processor is shown in FIG. 20), a memory 201, and a computer program 202 that is stored in the memory 201 and that can be run on the at least one processor 200. When executing the computer program 202, the processor 200 implements steps in any one of the foregoing embodiments of the electrocardiosignal prediction method.

The terminal device 20 may be a computing device such as a desktop computer, a notebook computer, a palmtop computer, or a cloud server. The terminal device may include but is not limited to including the processor 200 and the memory 201. A person skilled in the art may understand that FIG. 20 is merely an example of the terminal device 20, and does not constitute a limitation on the terminal device 20. The terminal device may include more or fewer components than those shown in the figure, or some components may be combined, or different components may be used. For example, the terminal device may further include an input/output device, a network access device, or the like.

The processor 200 may be a central processing unit (Central Processing Unit, CPU). The processor 200 may alternatively be another general-purpose processor, a digital signal processor (Digital Signal Processor, DSP), an application-specific integrated circuit (Application Specific Integrated Circuit, ASIC), a field-programmable gate array (Field-Programmable Gate Array, FPGA) or another programmable logic device, a discrete gate or a transistor logic device, or a discrete hardware component. The general-purpose processor may be a microprocessor, or the processor may be any conventional processor, or the like.

In some embodiments, the memory 201 may be an internal storage unit of the terminal device 20, for example, a hard disk drive or an internal storage of the terminal device 20. In some other embodiments, the memory 201 may alternatively be an external storage device of the terminal device 20, for example, a removable hard disk, a smart media card (Smart Media Card, SMC), a secure digital (Secure Digital, SD) card, a flash card (Flash Card), or the like that is provided on the terminal device 20. Further, the memory 201 may alternatively include both the internal storage unit and the external storage device of the terminal device 20. The memory 201 is configured to store an operating system, an application, a boot loader (Boot Loader), data, another program, and the like, for example, program code of the computer program. The memory 201 may further be configured to temporarily store data that has been output or is to be output.

It should be noted that content such as information exchange and an execution process between the foregoing apparatuses/units is based on a same concept as that in the method embodiments of this application. For specific functions and technical effects of the content, refer to the method embodiments. Details are not described herein again.

A person skilled in the art may clearly understand that, for the purpose of convenient and brief description, division into the foregoing functional units or modules is merely used as an example for description. In an actual application, the foregoing functions may be allocated to different functional units or modules for implementation according to a requirement. That is, an inner structure of the apparatus is divided into different functional units or modules, to implement all or some of the functions described above. Functional units or modules in embodiments may be integrated into one processing unit, or each of the units may exist alone physically, or two or more units may be integrated into one unit. The integrated unit may be implemented in a form of hardware, or may be implemented in a form of a software functional unit. In addition, specific names of the functional units or modules are merely provided for distinguishing between the units or modules, but are not intended to limit the protection scope of this application. For a specific working process of the units or modules in the foregoing system, refer to a corresponding process in the foregoing method embodiments. Details are not described herein again.

An embodiment of this application further provides a network device. The network device includes at least one processor, a memory, and a computer program that is stored in the memory and that can run on the at least one processor. When executing the computer program, the processor implements steps in any one of the foregoing method embodiments.

An embodiment of this application further provides a computer-readable storage medium. The computer-readable storage medium stores a computer program. When the computer program is executed by a processor, steps in the foregoing method embodiments can be implemented.

An embodiment of this application provides a computer program product. When the computer program product is run on a mobile terminal, the mobile terminal is enabled to implement steps in the foregoing method embodiments when executing the computer program product.

When the integrated unit is implemented in the form of a software functional unit and is sold or used as an independent product, the integrated unit may be stored in a computer-readable storage medium. Based on such an understanding, in this application, all or some of the procedures of the method in the foregoing embodiments may be implemented by a computer program instructing related hardware. The computer program may be stored in a computer-readable storage medium. When the computer program is executed by a processor, steps in the foregoing method embodiments can be implemented. The computer program includes computer program code, and the computer program code may be in a source code form, an object code form, an executable file form, some intermediate forms, or the like. The computer-readable medium may include at least any entity or apparatus that can carry computer program code to a photographing apparatus/terminal device, a recording medium, a computer memory, a read-only memory (ROM, Read-Only Memory), a random access memory (RAM, Random Access Memory), an electrical carrier signal, a telecommunications signal, and a software distribution medium, for example, a USB flash drive, a removable hard disk, a magnetic disk, or an optical disk. In some jurisdictions, according to legislation and patent practice, a computer-readable medium cannot be an electrical carrier signal or a telecommunications signal.

In the foregoing embodiments, the description of all embodiments has respective focuses. For a part that is not described in detail in an embodiment, refer to related descriptions in other embodiments.

A person of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

In embodiments provided in this application, it should be understood that the disclosed apparatus/network device and method may be implemented in other manners. For example, the described apparatus/network device embodiment is merely an example. For example, the module or unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions in the embodiments.

The foregoing embodiments are merely intended to describe the technical solutions of this application, but are not to limit this application. Although this application is described in detail with reference to the foregoing embodiments, a person of ordinary skill in the art should understand that they may still make modifications to the technical solutions described in the foregoing embodiments or make equivalent replacements to some technical features thereof, without departing from the spirit and scope of the technical solutions of embodiments of this application, and these modifications and replacements shall fall within the protection scope of this application.

**Claims**

1. An electrocardiosignal prediction method, comprising:

   obtaining an electrocardiosignal of a target user;
   importing the electrocardiosignal into a preset atrial fibrillation signal classification model, to obtain a signal type, of the electrocardiosignal, output by the atrial fibrillation signal classification model, wherein the atrial fibrillation signal classification model is obtained through training with an atrial fibrillation patient being a model training sample; and
   calculating, based on the signal type of the electrocardiosignal, a risk level of an atrial fibrillation occurrence, to predict whether the target user is to have an atrial fibrillation attack.

2. The prediction method according to claim 1, wherein before the importing the electrocardiosignal into a preset atrial fibrillation signal classification model, to obtain a signal type, of the electrocardiosignal, output by the atrial fibrillation signal classification model, wherein the atrial fibrillation signal classification model is obtained through training with an atrial fibrillation patient being a model training sample, the method further comprises:

   obtaining a training signal set comprising a plurality of successively collected historical signals, wherein the training signal set comprises at least one atrial fibrillation signal;
   extracting a risk signal other than the atrial fibrillation signal from the training signal set; and
   performing training by using the risk signal in the training signal set, to obtain the atrial fibrillation signal classification model.

3. The prediction method according to claim 2, wherein the performing training by using the risk signal in the training signal set, to obtain the atrial fibrillation signal classification model comprises:

   determining, based on a time difference between a collect time of the risk signal and a trigger time of an associated atrial fibrillation signal, a signal type corresponding to each risk signal;
   calculating a feature value of the risk signal in each preset signal feature dimension, to obtain a signal feature parameter of the risk signal; and
   performing training based on the signal feature parameter and the signal type, to obtain the atrial fibrillation signal classification model.

4. The prediction method according to claim 2, wherein the obtaining a training signal set comprising a plurality of successively collected electrocardiosignals comprises:

   obtaining a motion parameter that is of a training user and that is obtained during collection of the historical signal;
   determining jitter duration of the historical signal;
   determining, based on the jitter duration and the motion parameter, whether the historical signal is a valid signal; and
   encapsulating all valid information based on an order of collect times of all valid signals, to obtain the training signal set.

5. The prediction method according to any one of claims 1 to 4, wherein the importing the electrocardiosignal into a preset atrial fibrillation signal classification model, to obtain a signal type, of the electrocardiosignal, output by the atrial fibrillation signal classification model, wherein the atrial fibrillation signal classification model is obtained through training with an atrial fibrillation patient being a model training sample comprises:

   determining a vital sign parameter of the target user based on the electrocardiosignal;
   adjusting a classification threshold of the atrial fibrillation signal classification model based on the vital sign parameter; and
   identifying the signal type of the electrocardiosignal by using an adjusted atrial fibrillation signal classification model.

6. The prediction method according to any one of claims 1 to 4, wherein the calculating, based on the signal type of the electrocardiosignal, a risk level of an atrial fibrillation occurrence, to predict whether the target user is to have an atrial fibrillation attack comprises:

   counting a quantity of electrocardiosignals that are of the target user and whose signal types are a risk type within a preset time period; and
   calculating an atrial fibrillation occurrence probability based on the signal quantity.

7. The prediction method according to any one of claims 1 to 4, wherein if an atrial fibrillation occurrence probability is greater than a preset probability threshold, after the calculating, based on the signal type of the electrocardiosignal, a risk level of an atrial fibrillation occurrence, to predict whether the target user is to have an atrial fibrillation attack, the method further comprises:

   determining an associated user preset by the target user, and sending warning information to a terminal of the associated user; and/or
   obtaining, based on current location information of the target user, an address of a hospital closest to a location in the location information; and
   generating a path for treatment based on the location information and the hospital address.

8. The prediction method according to any one of claims 1 to 4, wherein after the calculating, based on the signal type of the electrocardiosignal, a risk level of an atrial fibrillation occurrence, to predict whether the target user is to have an atrial fibrillation attack, the method further comprises: if a new electrocardiosignal is received, identifying a new signal type of the new electrocardiosignal by using the atrial fibrillation signal classification model;

   recalculating an atrial fibrillation occurrence probability of the target user based on the new signal type; and
   generating an atrial fibrillation probability curve based on all identified atrial fibrillation occurrence probabilities.

9. An electrocardiosignal prediction apparatus, comprising:

   an electrocardiosignal obtaining unit, configured to obtain an electrocardiosignal of a target user;
   a signal type identification unit, configured to import the electrocardiosignal into a preset atrial fibrillation signal classification model, to obtain a signal type, of the electrocardiosignal, output by the atrial fibrillation signal classification model, wherein the atrial fibrillation signal classification model is obtained through training with an atrial fibrillation patient being a model training sample; and
   an atrial fibrillation occurrence probability calculation unit, configured to calculate, based on the signal type of the electrocardiosignal, a risk level of an atrial fibrillation occurrence, to predict whether the target user is to have an atrial fibrillation attack.

10. A terminal device, comprising a memory, a processor, and a computer program that is stored in the memory and that is run on the processor, wherein when executing the computer program, the processor implements the method according to any one of claims 1 to 8.

11. A computer-readable storage medium, wherein the computer-readable storage medium stores a computer program, and when the computer program is executed by a processor, the method according to any one of claims 1 to 8 is implemented.

FIG. 1

240

220

210

230

Wearable
device

Mobile
terminal

Cloud server

Other electronic
devices

FIG. 2

330

310

320

Wearable device

Server

Mobile
terminal

FIG. 3

S401

Obtain an electrocardiosignal of a target user

S402

Import the electrocardiosignal into a preset atrial fibrillation signal classification model, to obtain a signal type, of the electrocardiosignal, output by the atrial fibrillation signal classification model

S403

Calculate, based on the signal type of the electrocardiosignal, a risk level of an atrial fibrillation occurrence, to predict whether the target user is to have an atrial fibrillation attack

FIG. 4

FIG. 5

FIG. 6

S701

Obtain a training signal set including a plurality of successively collected historical signals

S702

Extract a risk signal other than an atrial fibrillation signal from the training signal set

S703

Perform training by using the risk signal in the training signal set, to obtain an atrial fibrillation signal classification model

FIG. 7

FIG. 8

S901

Determine, based on a time difference between a collect time of a risk signal and a trigger time of an associated atrial fibrillation signal, a signal type corresponding to each risk signal

S902

Calculate a feature value of the risk signal in each preset signal feature dimension, to obtain a signal feature parameter of the risk signal

S903

Perform training based on the signal feature parameter and the signal type, to obtain the atrial fibrillation signal classification model

FIG. 9

FIG. 10

| | S1101 |
| --- | --- |
| Obtain a motion parameter that is of a training user and that is obtained during collection of a historical signal | |

| | S1102 |
| --- | --- |
| Determine jitter duration of the historical signal | |

| | S1103 |
| --- | --- |
| Determine, based on the jitter duration and the motion parameter, whether the historical signal is a valid signal | |

| | S1104 |
| --- | --- |
| Encapsulate all valid information based on an order of collect times of all valid signals, to obtain a training signal set | |

FIG. 11

S1201

Determine a vital sign parameter of a target user based on an electrocardiosignal

S1202

Adjust a classification threshold of an atrial fibrillation signal classification model based on the vital sign parameter

S1203

Identify a signal type of the electrocardiosignal by using an adjusted atrial fibrillation signal classification model

FIG. 12

S4031

Count a quantity of electrocardiosignals that are of a target user and whose signal types are a risk type within a preset time period

S4032

Calculate an atrial fibrillation occurrence probability based on the signal quantity

FIG. 13

S4011

Determine an associated user preset by a target user, and send warning information to a terminal of the associated user

S4012

Obtain, based on current location information of the target user, an address of a hospital closest to a location in the location information

S4013

Generate a path for treatment based on the location information and the hospital address

FIG. 14

88

Contact an associated user

Terminal device of a user A

Note:
A current atrial fibrillation occurrence probability of the user A is 88%. Pay attention to a physical condition of the user A.

Terminal of the associated user

FIG. 15

Query a path to a hospital

Hospital address

Current location

FIG. 16

S1701

If a new electrocardiosignal is received, identify a new signal type of the new electrocardiosignal by using an atrial fibrillation signal classification model

S1702

Recalculate an atrial fibrillation occurrence probability of a target user based on the new signal type

S1703

Generate an atrial fibrillation probability curve based on all identified atrial fibrillation occurrence probabilities

FIG. 17

Atrial fibrillation occurrence probability

Atrial
fibrillation
occurrence
probability

View a probability trend

FIG. 18

191

| Electrocardiosignal obtaining unit |
| --- |

192

| Signal type identification unit |
| --- |

193

| Atrial fibrillation occurrence probability calculation unit |
| --- |

FIG. 19

20

200

| Memory | Processor |
| --- | --- |
| 201 | |
| Computer program | |

202

Terminal device

FIG. 20

## INTERNATIONAL SEARCH REPORT

| | International application No. |
| --- | --- |
| | **PCT/CN2020/136538** |

**A. CLASSIFICATION OF SUBJECT MATTER**

A61B 5/0205(2006.01)i

According to International Patent Classification (IPC) or to both national classification and IPC

**B. FIELDS SEARCHED**

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

CNKI, CNPAT, WPI, EPODOC: 华为, 李露平, 陈茂林, 韩羽佳, 贾淼, 郭光明, 房颤, 评估, 预测, 风险, 模型, 建模, atria, atrium, fibrillation, sort, estimate, model

**C. DOCUMENTS CONSIDERED TO BE RELEVANT**

| Category* | Citation of document, with indication, where appropriate, of the relevant passages | Relevant to claim No. |
| --- | --- | --- |
| X | CN 110403600 A (WUHAN HAIXINGTONG TECHNOLOGY CO., LTD.) 05 November 2019 (2019-11-05) description, paragraphs [0046]-[0076] | 1, 2, 5-11 |
| Y | CN 110403600 A (WUHAN HAIXINGTONG TECHNOLOGY CO., LTD.) 05 November 2019 (2019-11-05) description, paragraphs [0046]-[0076] | 3-4 |
| Y | CN 110573211 A (CARDIAC PACEMAKERS, INC.) 13 December 2019 (2019-12-13) description, paragraphs [0076]-[0077] | 3 |
| Y | CN 109745041 A (SAMSUNG ELECTRONICS CO., LTD.) 14 May 2019 (2019-05-14) claims 1-9 | 4 |
| X | CN 110547792 A (PING AN TECHNOLOGY (SHENZHEN) CO., LTD.) 10 December 2019 (2019-12-10) description, paragraphs [0006]-[0029] | 1, 2, 5-11 |
| A | US 2019125273 A1 (MEDTRONIC, INC.) 02 May 2019 (2019-05-02) entire document | 1-11 |

☑ Further documents are listed in the continuation of Box C.        ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **23 February 2021** | **11 March 2021** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/136538** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2016022162 A1 (SINGAPORE HEALTH SERVICES PTE. LTD.) 28 January 2016 (2016-01-28)<br>      entire document | 1-11 |

Form PCT/ISA/210 (second sheet) (January 2015)

**INTERNATIONAL SEARCH REPORT**
**Information on patent family members**

| International application No. |
| --- |
| **PCT/CN2020/136538** |

| Patent document cited in search report | | | Publication date (day/month/year) | Patent family member(s) | | | Publication date (day/month/year) |
| --- | --- | --- | --- | --- | --- | --- | --- |
| CN | 110403600 | A | 05 November 2019 | None | | | |
| CN | 110573211 | A | 13 December 2019 | EP | 3592419 | A1 | 15 January 2020 |
| | | | | US | 2018256053 | A1 | 13 September 2018 |
| | | | | WO | 2018164840 | A1 | 13 September 2018 |
| | | | | JP | 2020509840 | A | 02 April 2020 |
| CN | 109745041 | A | 14 May 2019 | TW | 201918222 | A | 16 May 2019 |
| | | | | US | 2019133468 | A1 | 09 May 2019 |
| | | | | KR | 20190050693 | A | 13 May 2019 |
| | | | | JP | 2019084343 | A | 06 June 2019 |
| | | | | DE | 102018117484 | A1 | 09 May 2019 |
| CN | 110547792 | A | 10 December 2019 | None | | | |
| US | 2019125273 | A1 | 02 May 2019 | US | 10702213 | B2 | 07 July 2020 |
| US | 2016022162 | A1 | 28 January 2016 | EP | 2964077 | A1 | 13 January 2016 |
| | | | | SG | 11201507172 X | A | 29 October 2015 |
| | | | | US | 9775533 | B2 | 03 October 2017 |
| | | | | US | 2014257122 | A1 | 11 September 2014 |
| | | | | US | 10299689 | B2 | 28 May 2019 |
| | | | | CN | 105228508 | A | 06 January 2016 |
| | | | | WO | 2014137295 | A1 | 12 September 2014 |
| | | | | CN | 105228508 | B | 03 April 2020 |
| | | | | SG | 11201507172 | B | 21 April 2017 |

Form PCT/ISA/210 (patent family annex) (January 2015)

**REFERENCES CITED IN THE DESCRIPTION**

**Patent documents cited in the description**

- CN 201911307688